# EUROPÄISCHE PATENTSCHRIFT

(11) **EP 1 754 465 B1**
(45) Veröffentlichungstag und Bekanntmachung des Hinweises auf die Patenterteilung: **05.10.2011**
(21) Anmeldenummer: 06016385.4
(22) Anmeldetag: 05.08.2006
(51) Int. Cl.: A61K 6/083, C08F 4/40

(54) **2-Komponenten-Initiatorsystem (aminfrei) mit Lagerstabilität und besonderer Eignung für acide Systeme**
2-component initiator system (amine free) with storage stability and especially suitable for acidic systems
Système d'amorçage à deux composants (sans amines) avec stabilité de stockage et notamment adapté aux systèmes acidiques

(30) Priorität: 19.08.2005 DE 102005039590
(43) Veröffentlichungstag der Anmeldung: 21.02.2007
(73) Patentinhaber: Heraeus Kulzer GmbH, 63450 Hanau (DE)
(72) Erfinder: Utterodt, Andreas, Dr., 61267 Neu-Anspach (DE); Hennen, Heike, 50354 Hürth (DE)
(74) Vertreter: Kühn, Hans-Christian

(56) Entgegenhaltungen:
- EP-A- 1 502 569
- EP-A2- 0 732 098
- EP-A2- 0 951 894
- GB-A- 1 177 879
- GB-A- 2 075 035
- US-A1- 2003 134 933
- DATABASE WPI Week 199007 Derwent Publications Ltd., London, GB; AN 1990-048041 XP002413141 & JP 02 000681 A (DENKI KAGAKU KOGYO KK) 5. Januar 1990 (1990-01-05)
- DATABASE WPI Week 198011 Derwent Publications Ltd., London, GB; AN 1980-19414C XP002413142 & JP 55 016018 A (DENKI KAGAKU KOGYO KK) 4. Februar 1980 (1980-02-04)

## Beschreibung

Die Erfindung betrifft ein neuartiges, 2-Komponenten-Initiatorsystem mit Beschleuniger zur Härtung von polymerisierbaren Materialien.

Zweikomponenten-Initiatorsysteme zur Härtung von polymerisierbaren Materialien sind bekannt:
1. Benzoylperoxid/tert.-aromatisches Amin: Dibenzoylperoxid zeigt die höchste Thermostabilität unter den mit aminen initiierbaren Peroxiden. Eine dauerhafte Erwärmung der Zubereitungen führt trotzdem zu einer bald eintretenden spontanen Aushärtung. Zum Nachteil für dentale Anwendungen wird auch die langsam eintretende Oxidation der Amine, die zu einer gelb-braunen Verfärbung führt und die Esthetik beeinträchtigt. In einer sauren Umgebung, wie sie in Adhäsiven notwendig ist, wird das Amin in einer Säure-Base-Reaktion umgehend protoniert und damit deaktiviert. Unter solchen Bedingungen kann das 2K-System nicht eingesetzt werden.
2. Barbitursäure-Derivate: In dentalen Anwendungen wird gern das Barbitursäure-Initiatorsystem verwendet, weil es nicht zu einer stärkeren nachträglichen Verfärbung kommt. Die zweite Komponente enthält Kupfer- und Chloridionen, die den Zerfall der Barbitursäure katalysieren. Da kein Amin enthalten ist, kann das System auch unter aciden Bedingungen eingesetzt werden. Aufgrund der geringen Thermostabilität müssen die Produkte stets kühl gelagert werden. Die Initiatorkomponente darf keine reaktiven, vernetzenden Inhaltsstoffe enthalten, da es zur spontanen Polymerisation kommt.
3. Cumenhydroperoxid/Acetylthioharnstoff: Ein endodontisches Versiegelungs-System wurde in der US 2003/0134933 (Pentron) beschrieben. Es beinhaltet Cumenhydroperoxid und Acetylthioharnstoff in größeren Mengen (1-10%) und geht möglicherweise auf die Beschreibung eines Klebstoffes zurück JP 58219281A (Mitsubishi). Es wurde eine sehr hohe Thermostabilität gegenüber dem gebräuchlichen System beschrieben. Die Nachstellung der Zusammensetzung zeigte aber eine unbrauchbar langsame Aushärtungskinetik im unteren Konzentrationsbereich. An der unteren Grenze der Beschreibung (jeweils 1 %) blieb die Polymerisation völlig aus.
4. Cumenhydroperoxid/Beschleuniger: Ein Klebstoff nach DE 195 01 933 (Henkel) enthält als Initiator ein Hydroperoxid und als Beschleuniger eine der nachfolgenden Verbindungen: Sulfimide, Hydrazinderivate, tert.-Amine und Kupfer-Salz oder -Komplexe. Zusätzlich können Sikkative zur Beschleunigung der Vernetzung enthalten sein. Eine hohe Reaktivität wird in einzelnen Zusammensetzungen erreicht, allerdings sind diese durch die Metallsalze stark gefärbt, vergilben stark oder zeigen eine ungenügende Lagerstabilität.
5. Peroxid/Metallverbindung: Eine polymerisierbare Zusammensetzung mit einem thermostabilen Peroxid wird in der DE 696 21 500 (Dentsply) beschrieben. Das polymerisierbare System kann neben Cumenhydroperoxid auch eine Metallverbindung als Thioharnstoffkomplex enthalten. Als Metallverbindungen sind unter anderem Kupferverbindungen beschrieben. Es muss auch eine Säure und ein Amin enthalten sein. In den Ansprüchen wurde speziell ein Pulver-Flüssigkeits-System beschrieben. Obgleich das Initiatorsystem wie beschrieben in einer säurehaltigen Umgebung offenbar funktionsfähig ist, kann das enthaltene Amin zu der typischen Verfärbung solcher Produkte führen.
6. In EP 1 479 364A1 werden aminfreie Zweikomponenten Dentalzusammensetzungen beschrieben, die ein Thioharnstoff-Hydroperoxid-Initiatorsystem umfassen.

Gemäß DE3116132A1verbessern katalytische Mengen von Kupferverbindungen die Initiatorwirkung solcher Thioharnstoff-Hydroperoxid-Initiatorsysteme deutlich.

Aufgabe ist demgegenüber die Entwicklung eines verbesserten Systems mit kurzen Inhibierungszeiten und niedrigen Gehalten der Initiatorkomponenten, wobei kein Amin enthalten ist, um Verfärbungen zu unterbinden, und die Funktionsfähigkeit unter sauren Bedingungen gewährleistet ist.

Die Aufgabe wird daher durch Zusammensetzungen nach Anspruch 1 gelöst. Die Unteransprüche geben vorteilhafte Ausgestaltungen an.

Als besonders geeignet hat sich die Kombination der Redoxpartner Cumenhydroperoxid und Acetylthioharnstoff in Gegenwart von Kupferverbindungen erwiesen. Die Komponenten sind allein und in reaktiven Monomeren auch bei hohen Temperaturen um 50°C über mehrere Monate lagerfähig. Die geringen Kupferanteile (< 0,1%) beschleunigen den initialen Redoxprozess so stark, dass eine Konzentration von 1% oder weniger der Redoxpartner ausreichend ist. Da kein Amin enthalten ist, wurde keine nachträgliche Farbänderung gefunden. Die schwache gelbgrüne Erscheinung des Polymers ist von der Kupferkonzentration abhängig und bleibt unverändert. Die Funktionsfähigkeit des 2K-Initiatorsystems wurde durch anwesende Säuren nicht beeinträchtigt.

### Das System hat folgende Vorteile

a) Die Zusammensetzung stellt ein 2K-Initiatorsystem mit sehr hoher Lagerstabilität dar. Es zeigt keine nachträgliche Verfärbung und wird durch Säuren nicht beeinträchtigt. Durch die Variation des Kupfergehaltes ist die Inhibierungszeit sehr variabel einstellbar. Es wurden zahlreiche Versuche zu verschiedenen Kombinationen durchgeführt und die jeweilige Inhibierungszeit bestimmt. Die Konzentrationen von Cumenhydroperoxid, Acetylthioharnstoff und Kupfersalz wurden variiert. Es wurden zwei Kupfersalze (Acetylacetonat, Naphthenat) geprüft, wobei keine Reaktivitätsunterschiede sichtbar wurden. Die Inhibierungszeiten wurden unter aciden Bedingungen durch Kombination mit Phosphorsäureester (2%, 5%), 4-META (15%) und Acrylsäure (5%) bestimmt. Die Lagerstabilitäten aller Zubereitungen wurde bei 50 °C über einen Zeitraum bis zu 3 Monaten verfolgt. Der Einfluss des Kupfergehaltes auf die Färbung des Polymers wurde bestimmt. Die Farbstabilität wurde überprüft.
b) Das Initiatorsystem ist universell einsetzbar für alle radikalisch polymerisierbaren Systeme. Zur Herstellung von medizinischen oder dentalen Kunststoffen ist die sehr gute Lagerstabilität und die geringe Verfärbungsneigung besonders vorteilhaft. Die Möglichkeit zur Kombination mit aciden Komponenten kann neue Einsatzgebiete erschließen (Adhäsive, selbstätzende Zubereitungen).
c) Gegenüber dem konventionellen 2K-System bestehend aus Dibenzoylperoxid und einem tertiären aromatischen Amin zeichnet es sich durch eine deutlich bessere Lagerstabilität aus, arbeitet auch unter aciden Bedingungen und zeigt eine verbesserte Farbstabilität.

Die Zusammensetzung beschreibt ein Redox-System und enthält im aktivierten Zustand ein Hydroperoxid als Oxidationsmittel und ein Thioharnstoff-Derivat als Reduktionsmittel. Ein so beschriebenes Initiatorsystem ist bereits bekannt aus den Patentschriften JP 58219281, US 2003/0134933, US 2003/0166740 und EP 1 479364.

Unerwartet wurde als Beschleuniger mit deutlich verbesserter Initiierung ein Zusatz von löslichen Kupferverbindungen gefunden. Der Zusatz von Säuren beeinträchtigt das System nicht bzw. kann sogar zu einer weiteren Steigerung der Initiierungsaktivität führen. Durch den Zusatz von Kupferverbindungen kann die Konzentration der reizenden Hydroperoxidkomponente und auch der Anteil des Thioharnstoff-Derivates verringert werden, und dennoch eine geeignete Inhibierungszeit (Verarbeitungszeit) erreicht werden. Die Lagerstabilität des 2K-Initiatorsystems wird durch einen Anteil der Kupferverbindung nicht nachteilig verändert, wie in Versuchen gezeigt werden konnte. Die schwach gelbliche Eigenfärbung der Polymere blieb dauerhaft unverändert.

Die erfindungsgemäße Zusammensetzung des 2K-Initiatorsystems enthält in einer Komponente ein organisches Hydroperoxid, wobei die Verbindung auch mehr als eine Hydroperoxid-Gruppe enthalten kann. Geeignet sind neben weiteren Verbindungen t-Butylhydroperoxid, t-Amylhydroperoxid, Benzenhydroperoxid, Pinanhydroperoxid, 1,1,3,3-Tetramethylbutylhydroperoxid, 5-Phenyl-4-pentenylhydroperoxid, p-Diisopropylbenzenhydroperoxid, besonders geeignet ist iso-Propylbenzenhydroperoxid.

In einer zweiten Komponente ist ein Thioharnstoffderivat und wenigstens eine Kupferverbindung enthalten. Beispiele solcher Thioharnstoff-Derivate sind:
1-(1,1-DIOXO-TETRAHYDRO-THIOPHEN-3-YL)-1-METHYL-3-PHENYL-THIOHARNSTOFF
1-(1,2-DIPHENYLETHYL)-3-(2,4-XYLYL)-2-THIOHARNSTOFF
1-(1,2-DIPHENYLETHYL)-3-(4-ETHOXYPHENYL)-2-THIOHARNSTOFF
1-(1,2-DIPHENYLETHYL)-3-(ORTHO-TOLYL)-2-THIOHARNSTOFF
1-(1,2-DIPHENYLETHYL)-3-PHENYL-2-THIOHARNSTOFF
1,1'-(3,3'-DIMETHYLBIPHENYL-4,4'-DIYL)BIS(3-(2-METHYLPROPYL)-2-THIOHARNSTOFF)
1,1,3-TRIPHENYL-2-THIOHARNSTOFF
1,1'-(4,5-DIMETHYL-1,2-PHENYLENE)BIS(3-PHENYL-2-THIOHARNSTOFF)
1-(1,5-DI-ME-3-OXO-2-PH-2,3-DIHYDRO-1H-PYRAZOL-4-YL)-3-(2-METHYL-ALLYL)-THIOHARNSTOFF
1-(1,5-DI-ME-3-OXO-2-PH-2,3-DIHYDRO-1H-PYRAZOL-4-YL)-3-(3-TRI-F-ME-PH)-THIOHARNSTOFF
1-(1,5-DIMETHYL-3-OXO-2-PHENYL-2,3-DIHYDRO-1 H-PYRAZOL-4-YL)-3-PHENYLTHIOHARNSTOFF
1,1-BIS-(2-HYDROXY-ETHYL)-3-PHENYL-THIOHARNSTOFF
1,1-DIALLYL-3-(3-CHLORO-BENZO(B)THIOPHEN E-2-CARBONYL)-THIOHARNSTOFF
1,1-DIALLYL-3-(4-NITROPHENYL)-2-THIOHARNSTOFF
1,1-DIALLYL-3-PHENYL-2-THIOHARNSTOFF
1,1-DIBENZYL-3-(2,4-DICHLORO-BENZOYL)-THIOHARNSTOFF
1,1-DIBENZYL-3-(2-(TRIFLUOROMETHYL)PHENYL)-2-THIOHARNSTOFF
1,1-DIBENZYL-3-PHENYL-2-THIOHARNSTOFF
1,1-DIBUTYL-3-PHENYL-2-THIOHARNSTOFF
1,1-DIETHYL-3-PHENYL-2-THIOHARNSTOFF
1,1-DIISOBUTYL-3-PHENYL-2-THIOHARNSTOFF
1,1-DIISOPROPYL-3-PHENYL-2-THIOHARNSTOFF
1,1-DIMETHYL-3-(2,6-XYLYL)-2-THIOHARNSTOFF
1,1-DIMETHYL-3-(2-METHOXYPHENYL)-2-THIOHARNSTOFF
1,1-DIMETHYL-3-(3,4-XYLYL)-2-THIOHARNSTOFF
1,1-DIMETHYL-3-(4-ETHOXYPHENYL)-2-THIOHARNSTOFF
1,1-DIMETHYL-3-(4-METHOXYPHENYL)-2-THIOHARNSTOFF
1,1-DIMETHYL-3-(ALPHA-(METHYLIMINO)BENZYL)-2-THIOHARNSTOFF
1,1-DIMETHYL-3-(META-TOLYL)-2-THIOHARNSTOFF
1,1-DIMETHYL-3-(ORTHO-TOLYL)-2-THIOHARNSTOFF
1,1-DIMETHYL-3-(PARA-TOLYL)-2-THIOHARNSTOFF
1,1-DIMETHYL-3-PHENYL-2-THIOHARNSTOFF
1,1-DIPROPYL-3-PHENYL-2-THIOHARNSTOFF
1-(1-ETHYL-3-PIPERIDINYL)-3-PHENYL-2-THIOHARNSTOFF
1-(1-NAPHTHYL)-2-THIOHARNSTOFF
1-(1-NAPHTHYL)-3-(2-PHENOXYPROPIONYL)-2-THIOHARNSTOFF
1,1-PENTAMETHYLENE-3-PHENYL-2-THIOHARNSTOFF
1-(2,2-DIMETHYL-PROPYL)-3-(2-FLUORO-PHENYL)-THIOHARNSTOFF
1-(2-(2-HYDROXY-ETHOXY)-ETHYL)-3-PHENYL-THIOHARNSTOFF
1-(2,4,6-TRIBROMOPHENYL)-2-THIOHARNSTOFF
1-(2,4-DICHLOROBENZOYL)-3-(1-NAPHTHYL)-2-THIOHARNSTOFF
1-(2,4-DICHLOROBENZOYL)-3-(7-HYDROXY-1-NAPHTHYL)-2-THIOHARNSTOFF
1-(2,4-DICHLOROPHENYL)-2-THIOHARNSTOFF
1-(2,4-DIFLUOROPHENYL)-3-(2-FLUOROPHENYL)-2-THIOHARNSTOFF
1-(2,4-DIFLUOROPHENYL)-3-(4-PHENOXYPHENYL)-2-THIOHARNSTOFF
1-(2,4-DIFLUOROPHENYL)-3-ETHYL-2-THIOHARNSTOFF
1-(2,5-DICHLOROPHENYL)-3-(2-PHENOXYPROPIONYL)-2-THIOHARNSTOFF
1-(2,5-DICHLOROPHENYL)-3-DODECANOYL-2-THIOHARNSTOFF
1-(2,5-DICHLOROPHENYL)-3-PHENYL-2-THIOHARNSTOFF

1-(2,5-DIMETHOXYPHENYL)-3-(3-NITROBENZOYL)-2-THIOHARNSTOFF
1-(2,5-DIMETHOXYPHENYL)-3-DODECANOYL-2-THIOHARNSTOFF
1-(2,5-DIMETHOXYPHENYL)-3-METHYL-2-THIOHARNSTOFF
1-(2,5-DIMETHOXYPHENYL)-3-PHENYL-2-THIOHARNSTOFF
1-(2,5-DIMETHOXYPHENYL)-3-PROPYL-2-THIOHARNSTOFF
1-(2,5-DIMETHYLMORPHOLINO)-3-PHENYL-2-THIOHARNSTOFF
1-(2,6-DIETHYLPHENYL)-3-(4-HYDROXYPHENYL)-2-THIOHARNSTOFF
1-(2,6-XYLYL)-2-THIOHARNSTOFF
1-(2-BROMO-4-METHYL-PHENYL)-3-PHENYL-THIOHARNSTOFF
1-(2-Bromophenyl)-2-THIOHARNSTOFF
1-(2-CARBOXYPHENYL)-3-PHENYL-2-THIOHARNSTOFF
1-(2-CHLORO-4-METHOXYPHENYL)-3-CYCLOHEXYL-2-THIOHARNSTOFF
1-(2-CHLORO-4-NITROPHENYL)-3-(3,4-DICHLOROBENZOYL)-2-THIOHARNSTOFF
1-(2-CHLORO-4-NITROPHENYL)-3-ETHYL-2-THIOHARNSTOFF
1-(2-CHLORO-5-NITROPHENYL)-3-DODECANOYL-2-THIOHARNSTOFF
1-(2-CHLOROBENZOYL)-3-(2,4-DIFLUOROPHENYL)-2-THIOHARNSTOFF
1-(2-CHLOROBENZOYL)-3-(2,4-DIMETHOXYPHENYL)-2-THIOHARNSTOFF
1-(2-CHLOROBENZOYL)-3-(2-FLUOROPHENYL)-2-THIOHARNSTOFF
1-(2-CHLOROBENZOYL)-3-(2-METHOXY-5-METHYLPHENYL)-2-THIOHARNSTOFF
1-(2-CHLOROBENZOYL)-3-(3,4-DICHLOROPHENYL)-2-THIOHARNSTOFF
1-(2-CHLOROBENZOYL)-3-(3-CHLOROPHENYL)-2-THIOHARNSTOFF
1-(2-CHLOROBENZOYL)-3-(4-NITROPHENYL)-2-THIOHARNSTOFF
1-(2-CHLOROBENZOYL)-3-(5-CHLORO-2-METHOXYPHENYL)-2-THIOHARNSTOFF
1-(2-CHLOROBENZYL)-3-CYCLOHEXYL-1-METHYL-2-THIOHARNSTOFF
1-(2-CHLOROPHENYL)-3-(2,4-DICHLOROBENZOYL)-2-THIOHARNSTOFF
1-(2-CHLOROPHENYL)-3-DODECANOYL-2-THIOHARNSTOFF
1-(2-CHLOROPHENYL)-3-PHENYL-2-THIOHARNSTOFF
1-(2-ETHYLPHENYL)-3-METHYL-2-THIOHARNSTOFF
1-(2-FLUOROBENZOYL)-3-(4-ETHOXYPHENYL)-2-THIOHARNSTOFF
1-(2-FLUOROBENZOYL)-3-(4-FLUOROPHENYL)-2-THIOHARNSTOFF
1-(2-(HEXADECYLTHIO)PHENYL)-2-THIOHARNSTOFF
1-(2-HYDROXY-1-PHENYL-ETHYL)-3-PHENYL-THIOHARNSTOFF
1-(2-HYDROXY-CYCLOHEXYL)-3-PHENYL-THIOHARNSTOFF
1-(2-HYDROXYETHYL)-3-(2,4-XYLYL)-2-THIOHARNSTOFF
1-(2-HYDROXYETHYL)-3-PHENYL-2-THIOHARNSTOFF
1-(2-METHOXY-5-METHYLPHENYL)-1-METHYL-3-(2-NAPHTHYL)-2-THIOHARNSTOFF
1-(2-METHOXY-PHENYL)-3-(2-METHYL-BENZOYL)-THIOHARNSTOFF
1-(2-METHOXYPHENYL)-2-THIOHARNSTOFF
1-(2-Methoxyphenyl)-2-THIOHARNSTOFF
1-(2-METHYL-2-MORPHOLIN-4-YL-PROPYL)-3-PHENYL-THIOHARNSTOFF
1-(2-METHYL-ALLYL)-3-(6-(3-(2-METHYL-ALLYL)-THIOUREIDO)-PYRIDIN-2-YL)-THIOHARNSTOFF
1-(2-METHYL-BENZOYL)-3-P-TOLYL-THIOHARNSTOFF
1-(2-METHYL-BENZOYL)-3-PHENYL-THIOHARNSTOFF
1-(2-METHYL-BENZOYL)-3-PYRIMIDIN-2-YL-THIOHARNSTOFF
1-(2-MORPHOLINOETHYL)-3-PHENYL-2-THIOHARNSTOFF
1-(2-NAPHTHYL)-3-PHENYL-2-THIOHARNSTOFF
1-(2-NITROPHENYL)-3-PHENYL-2-THIOHARNSTOFF
1-(2-PYRIDYL)-3-(3,4-XYLYL)-2-THIOHARNSTOFF
1-(2-PYRIDYL)-3-(ORTHO-TOLYL)-2-THIOHARNSTOFF
1-(3-(2-MERCAPTO-ETHYL)-3H-BENZOTHIAZOL-2-YLIDENE)-2-ME-ISOTHIOHARNSTOFF, HYDRIODIDE
1-(3,4-DIBROMOPHENYL)-3-PHENYL-2-THIOHARNSTOFF
1-(3,4-DICHLOROBENZOYL)-3-(2,4-DIFLUOROPHENYL)-2-THIOHARNSTOFF
1-(3,4-DICHLOROBENZOYL)-3-(2-FLUOROPHENYL)-2-THIOHARNSTOFF
1-(3,4-DICHLOROBENZOYL)-3-(2-THIAZOLYL)-2-THIOHARNSTOFF
1-(3,4-DICHLOROBENZOYL)-3-(3,4-DICHLOROPHENYL)-2-THIOHARNSTOFF
1-(3,4-DICHLOROBENZOYL)-3-(4-ETHOXYPHENYL)-2-THIOHARNSTOFF
1-(3,4-DICHLOROBENZOYL)-3-(4-NITROPHENYL)-2-THIOHARNSTOFF
1-(3,4-DICHLOROBENZOYL)-3-(4-SULFAMOYL)PHENYL-2-THIOHARNSTOFF
1-(3,4-Dichlorophenyl)-2-THIOHARNSTOFF
1-(3,4-DICHLOROPHENYL)-3-(3,5-DINITROBENZOYL)-2-THIOHARNSTOFF
1-(3,4-DICHLOROPHENYL)-3-(4-SULFAMOYLPHENYL)-2-THIOHARNSTOFF
1-(3,4-DICHLOROPHENYL)-3-(DIPHENYLMETHYL)-2-THIOHARNSTOFF
1-(3,4-DICHLOROPHENYL)-3-PHENYL-2-THIOHARNSTOFF
1-(3,4-DIMETHOXY-BENZYL)-3-PHENYL-THIOHARNSTOFF
1-(3,4-DIMETHOXYPHENYL)-2-THIOHARNSTOFF
1-(3,5-DICHLOROBENZYL)-3-PHENYL-2-THIOHARNSTOFF
1-(3,5-DICHLOROPHENYL)-2-THIOHARNSTOFF
1-(3-ACETYLPHENYL)-3-ALLYL-2-THIOHARNSTOFF
1-(3-ACETYLPHENYL)-3-ETHYL-2-THIOHARNSTOFF
1,3-BIS(2,6-DICHLOROPHENYL)-2-THIOHARNSTOFF
1,3-BIS(2-CHLOROPHENYL)-2-THIOHARNSTOFF
1,3-BIS(2-FLUOROPHENYL)-2-THIOHARNSTOFF
1,3-BIS-(2-METHOXYPHENYL)-2-THIOHARNSTOFF
1,3-BIS(3-ACETYLPHENYL)-2-THIOHARNSTOFF
1,3-BIS(3-BROMOPHENYL)-2-THIOHARNSTOFF
1,3-BIS(3-CYANOPHENYL)-2-THIOHARNSTOFF
1,3-BIS(3-IODOPHENYL)-2-THIOHARNSTOFF
1,3-BIS(3-METHOXYPHENYL)-2-THIOHARNSTOFF
1,3-BIS(3-NITROPHENYL)-2-THIOHARNSTOFF
1,3-Bis(3-pyridylmethyl)-2-THIOHARNSTOFF
1,3-BIS(4-BROMOPHENYL)-2-THIOHARNSTOFF
1,3-BIS(4-CHLOROPHENYL)-2-THIOHARNSTOFF
1,3-BIS(4-CYANOPHENYL)-2-THIOHARNSTOFF
1,3-BIS(4-(DIMETHYLAMINO)PHENYL)-2-THIOHARNSTOFF
1-(3-BROMO-PHENYL)-3-(2-METHYL-BENZOYL)-THIOHARNSTOFF
1-(3-BROMO-PHENYL)-3-(NAPHTHALENE-1-CARBONYL)-THIOHARNSTOFF
1-(3-BROMOBENZOYL)-3-(3-CHLOROPHENYL)-2-THIOHARNSTOFF
1-(3-BUTOXYPROPYL)-3-PHENYL-2-THIOHARNSTOFF
1-(3-Carboxyphenyl)-2-THIOHARNSTOFF
1-(3-CHLORO-2-METHYL-PHENYL)-3-(3,4,5-TRIMETHOXY-BENZOYL)-THIOHARNSTOFF
1-(3-CHLORO-2-METHYL-PHENYL)-3-(4-FLUORO-BENZOYL)-THIOHARNSTOFF
1-(3-CHLORO-BENZO(B)THIOPHENE-2-CARBONYL)-3-(4-NITRO-PHENYL)-THIOHARNSTOFF
1-(3-CHLOROBENZYL)-3-PHENYL-2-THIOHARNSTOFF
1-(3-CHLOROPHENYL)-2-THIOHARNSTOFF
1-(3-CHLOROPHENYL)-3-(2-FLUOROBENZOYL)-2-THIOHARNSTOFF
1-(3-CHLOROPHENYL)-3-(3,4-DICHLOROBENZOYL)-2-THIOHARNSTOFF
1-(3-CHLOROPHENYL)-3-DODECANOYL-2-THIOHARNSTOFF
1-(3-CHLOROPHENYL)-3-PHENYL-2-THIOHARNSTOFF
1,3-DI-O-TOLYL-2-THIOHARNSTOFF
1,3-Di-*o*-tolyl-2-THIOHARNSTOFF
1,3-Di-*p*-tolyl-2-THIOHARNSTOFF
1,3-DI-TERT-BUTYL-2-THIOHARNSTOFF

1,3-DIALLYL-2-THIOHARNSTOFF
1,3-DIBENZYL-2-THIOHARNSTOFF
1-(3-(DIBUTYLAMINO)PROPYL)-3-PHENYL-2-THIOHARNSTOFF
1,3-DICYCLOHEXYL-2-THIOHARNSTOFF
1,3-DIDECYL-2-THIOHARNSTOFF
1,3-DIDODECYL-2-THIOHARNSTOFF
1,3-DIFURFURYL-2-THIOHARNSTOFF
1,3-DIHEPTYL-2-THIOHARNSTOFF
1,3-DIHEXADECYL-2-THIOHARNSTOFF
1,3-DIHEXYL-2-THIOHARNSTOFF
1,3-Diisopropyl-2-THIOHARNSTOFF
1,3-DIOCTYL-2-THIOHARNSTOFF
1,3-DIPROPYL-2-THIOHARNSTOFF
1,3-DITETRADECYL-2-THIOHARNSTOFF
1-(3-FLUOROPHENYL)-3-(3-NITROBENZOYL)-2-THIOHARNSTOFF
1-(3-FLUOROPHENYL)-3-METHYL-2-THIOHARNSTOFF
1-(3-HYDROXYPHENYL)-3-(4-NITROPHENYL)-2-THIOHARNSTOFF
1-(3-HYDROXYPHENYL)-3-PHENYL-2-THIOHARNSTOFF
1-(3-METHOXY-PHENYL)-3-(2-METHYL-BENZOYL)-THIOHARNSTOFF
1-(3-METHOXY-PHENYL)-3-(NAPHTHALENE-1-CARBONYL)-THIOHARNSTOFF
1-(3-METHOXYPROPYL)-2-THIOHARNSTOFF
1-(3-NITRO-PHENYL)-3-(3-PHENYL-ACRYLOYL)-THIOHARNSTOFF
1-(3-NITROPHENYL)-3-PHENYL-2-THIOHARNSTOFF
1-(3-PHENYLPROPIONYL)-3-(2,3-XYLYL)-2-THIOHARNSTOFF
1-(3-PYRIDYL)-2-THIOHARNSTOFF
1-(3-(TRIFLUOROMETHYL)PHENYL)-2-THIOHARNSTOFF
1-(3-TRIFLUOROMETHYL-PHENYL)-3-(2,3,4-TRIMETHOXY-BENZOYL)-THIOHARNSTOFF
1-(3H-BENZOTHIAZOL-2-YLIDENE)-2-BENZYL-ISOTHIOHARNSTOFF
1-(4-ACETAMIDOPHENYL)-3-(2-BUTENOYL)-2-THIOHARNSTOFF
1-(4-ACETAMIDOPHENYL)-3-(2-PHENOXYACETYL)-2-THIOHARNSTOFF
1-(4-ACETAMIDOPHENYL)-3-ETHYL-2-THIOHARNSTOFF
1-(4-ACETYLPHENYL)-3-METHYL-2-THIOHARNSTOFF
1-(4-BROMO-PHENYL)-3-(2,4-DICHLORO-BENZOYL)-THIOHARNSTOFF
1-(4-CHLORO-2,5-DIMETHOXYPHENYL)-3-(4-NITROPHENYL)-2-THIOHARNSTOFF
1-(4-CHLORO-2,5-DIMETHOXYPHENYL)-3-METHYL-2-THIOHARNSTOFF
1-(4-CHLORO-2-METHYL-PHENYL)-3-(2-METHYL-3-PHENYL-ACRYLOYL)-THIOHARNSTOFF
1-(4-CHLORO-2-METHYLPHENYL)-3-DODECANOYL-2-THIOHARNSTOFF
1-(4-CHLORO-7-METHOXY-2-QUINOLYL)-3-PHENYL-2-THIOHARNSTOFF
1-(4-CHLORO-PHENYL)-3-(4-NITRO-BENZOYL)-THIOHARNSTOFF
1-(4-CHLORO-PHENYL)-3-(NAPHTHALENE-1-CARBONYL)-THIOHARNSTOFF
1-(4-CHLOROBENZYL)-1-METHYL-3-PHENYL-2-THIOHARNSTOFF
1-(4-CHLOROPHENYL)-2-THIOHARNSTOFF
1-(4-CHLOROPHENYL)-3-(2-PYRIDYL)-2-THIOHARNSTOFF
1-(4-CHLOROPHENYL)-3-(4-DIETHYLAMINO-ORTHO-TOLYL)-2-THIOHARNSTOFF javascript:submitRefinement('expand','200117788')1-(4-CHLOROPHENYL)-3-PHENYL-2-THIOHARNSTOFF
1-(4-DIFLUOROMETHOXY-2-FORMYL-PHENYL)-3-(1,2,2-TRIMETHYL-PROPYL)-THIOHARNSTOFF
1-(4-DIFLUOROMETHOXY-PHENYL)-3-(1,2,2-TRIMETHYL-PROPYL)-THIOHARNSTOFF
1-(4-DIFLUOROMETHYLSULFANYL-PHENYL)-3-(1,2,2-TRIMETHYL-PROPYL)-THIOHARNSTOFF
1-(4-(DIMETHYLAMINO)PHENYL)-3-(4-NITROPHENYL)-2-THIOHARNSTOFF
1-(4-(DIMETHYLAMINO)PHENYL)-3-DODECANOYL-2-THIOHARNSTOFF
1-(4-(DIMETHYLAMINO)PHENYL)-3-(PHENYL)-2-THIOHARNSTOFF
1-(4-ETHOXYCARBONYL)-3-ETHYL-2-THIOHARNSTOFF
1-(4-ETHOXYPHENYL)-3-(1-NAPHTHYLCARBONYL)-2-THIOHARNSTOFF
1-(4-ETHOXYPHENYL)-3-(2-PYRIDYL)-2-THIOHARNSTOFF
1-(4-ETHOXYPHENYL)-3-(3-NITROBENZOYL)-2-THIOHARNSTOFF
1-(4-ETHOXYPHENYL)-3-ETHYL-2-THIOHARNSTOFF
1-(4-ETHYLPHENYL)-3-PHENYL-2-THIOHARNSTOFF
1-(4-FLUOROPHENYL)-3-(2,4,6-TRIMETHYLPHENYL)-2-THIOHARNSTOFF
1-(4-FLUOROPHENYL)-3-(3-NITROBENZOYL)-2-THIOHARNSTOFF
1-(4-(HEXADECYLSULFONYL)-PHENYL)-2-THIOHARNSTOFF
1-(4-HYDROXYPHENYL)-3-PROPYL-2-THIOHARNSTOFF
1-(4-IODOPHENYL)-3-(3-PHENYLPROPIONYL)-2-THIOHARNSTOFF
1-(4-MEO-PH)-3-((4-MEO-PHENYL)-(TOLUENE-4-SULFONYLMETHYLIMINO)-METHYL)-THIOHARNSTOFF
1-(4-METHOXY-BENZYL)-3-PHENYL-THIOHARNSTOFF
1-(4-METHOXY-PHENYL)-3-(4-METHYL-BENZOYL)-THIOHARNSTOFF
1-(4-METHOXY-PHENYL)-3-PHENYL-THIOHARNSTOFF
1-(4-METHOXYPHENYL)-3-(2-PYRIDYL)-2-THIOHARNSTOFF
1-(4-METHOXYPHENYL)-3-(2-PYRIDYL)-2-THIOHARNSTOFF
1-(4-METHYLBENZYL)-3-PHENYL-2-THIOHARNSTOFF
1-(4-NITRO-BENZOYL)-3-PYRIDIN-2-YLMETHYL-THIOHARNSTOFF
1-(4-Nitrophenyl)-2-THIOHARNSTOFF
1-(4-NITROPHENYL)-3-(3-(TRIFLUOROMETHYL)PHENYL)-2-THIOHARNSTOFF
1-(4-NITROPHENYL)-3-PHENYL-2-THIOHARNSTOFF
1-(4-PYRIDYLMETHYL)-3-(2,6-XYLYL)-2-THIOHARNSTOFF
1-(4-TERT.-BUTYLPHENYL)-2-THIOHARNSTOFF
1-(4-TRIFLUOROMETHOXY-PHENYL)-3-(1,2,2-TRIMETHYL-PROPYL)-THIOHARNSTOFF
1-(4-TRIFLUOROMETHYLSULFANYL-PHENYL)-3-(1,2,2-TRIMETHYL-PROPYL)-THIOHARNSTOFF
1-(5-ANILINO-1,2,4-THIADIAZOL-3-YL)-3-PHENYL-2-THIOHARNSTOFF
1-(5-BROMO-3-METHYL-PYRIDIN-2-YL)-3-PHENYL-THIOHARNSTOFF
1-(5-CHLORO-2,4-DIMETHOXYPHENYL)-3-(2,4-DIFLUOROPHENYL)-2-THIOHARNSTOFF
1-(5-CHLORO-2,4-DIMETHOXYPHENYL)-3-(2,5-DIMETHOXYPHENYL)-2-THIOHARNSTOFF
1-(5-CHLORO-2,4-DIMETHOXYPHENYL)-3-(2-FLUOROPHENYL)-3-METHYL-2-THIOHARNSTOFF
1-(5-CHLORO-2,4-DIMETHOXYPHENYL)-3-METHYL-2-THIOHARNSTOFF
1-(5-CHLORO-2-METHOXYPHENYL)-3-(3,4-DICHLOROBENZOYL)-2-THIOHARNSTOFF
1-(5-CHLORO-2-METHOXYPHENYL)-3-PHENYL-2-THIOHARNSTOFF
1-(5-HYDROXY-1-NAPHTHYL)-3-PHENYL-2-THIOHARNSTOFF
1-ACETYL-3-PHENYL-2-THIOHARNSTOFF
1-ALLYL-3-(1,2-DIPHENYLETHYL)-2-THIOHARNSTOFF
1-ALLYL-3-(1,5-DIMETHYL-3-OXO-2-PHENYL-2,3-DIHYDRO-1H-PYRAZOL-4-YL)-THIOHARNSTOFF
1-ALLYL-3-(1-NAPHTHYL)-2-THIOHARNSTOFF
1-ALLYL-3-(1-NAPHTHYLMETHYL)-2-THIOHARNSTOFF
1-ALLYL-3-(2-(1-HYDROXYETHYL)PHENYL)-2-THIOHARNSTOFF
1-ALLYL-3-(2-ETHOXYPHENYL)-2-THIOHARNSTOFF
1-ALLYL-3-(3-CHLORO-2-METHYLPHENYL)-2-THIOHARNSTOFF
1-ALLYL-3-(3-HYDROXYPHENYL)-2-THIOHARNSTOFF
1-ALLYL-3-(4-CHLOROBENZYL)-2-THIOHARNSTOFF
1-ALLYL-3-(4-CHLOROPHENYL)-2-THIOHARNSTOFF

1-ALLYL-3-(4-HYDROXYPHENYL)-2-THIOHARNSTOFF
1-ALLYL-3-(DIPHENYLMETHYL)-2-THIOHARNSTOFF
1-ALLYL-3-MORPHOLIN-4-YL-THIOHARNSTOFF
1-ALLYL-3-O-TOLYL-THIOHARNSTOFF
1-ALLYL-3-OCTADECYL-2-THIOHARNSTOFF
1-ALLYL-3-PHENYL-2-THIOHARNSTOFF
1-(ALPHA-METHYLBENZYL)-3-(4-PYRIDYLMETHYL)-2-THIOHARNSTOFF
1-(ALPHA-METHYLBENZYL)-3-PHENYLTHIOHARNSTOFF
1-AMIDINO-2-THIOHARNSTOFF OXALATE
1-AMIDINO-3-(4-BROMOPHENYL)-2-THIOHARNSTOFF
1-AMIDINO-3-BENZOYL-2-THIOHARNSTOFF
1-AMIDINO-3-METHYL-2-THIOHARNSTOFF P-TOLUENESULFONATE
1-AMIDINO-3-(P-TOLYL)-2-THIOHARNSTOFF
1-AMIDINO-3-PROPYL-2-THIOHARNSTOFF
1-AMIDINO-3-PROPYL-2-THIOHARNSTOFF P-TOLUENESULFONATE
1-BENZOTHIAZOL-2-YL-3-PHENYL-THIOHARNSTOFF
1-BENZOYL-3-(1,5-DIMETHYL-3-OXO-2-PHENYL-2,3-DIHYDRO-1H-PYRAZOL-4-YL)-THIOHARNSTOFF
1-BENZOYL-3-(1,5-DIMETHYL-3-OXO-2-PHENYL-PYRAZOLIDIN-4-YL)-THIOHARNSTOFF
1-BENZOYL-3-(2,4,6-TRIMETHYL-PHENYL)-THIOHARNSTOFF
1-BENZOYL-3-(2,6-DICHLOROPHENYL)-2-THIOHARNSTOFF
1-BENZOYL-3-(2-CHLORO-BENZYL)-THIOHARNSTOFF
1-BENZOYL-3-(2-CHLOROPHENYL)-2-THIOHARNSTOFF
1-BENZOYL-3-(3,4-DIMETHOXYPHENETHYL)-2-THIOHARNSTOFF
1-BENZOYL-3-(4-DIMETHYLAMINO-PHENYL)-THIOHARNSTOFF
1-BENZOYL-3-(4-METHOXY-2-NITROPHENYL)-2-THIOHARNSTOFF
1-BENZOYL-3-(4-MORPHOLIN-4-YL-PHENYL)-THIOHARNSTOFF
1-BENZOYL-3-(4-NITROPHENYL)-2-THIOHARNSTOFF
1-BENZOYL-3-(4-OXO-THIAZOLIDIN-2-YLIDENE)-THIOHARNSTOFF
1-BENZOYL-3-(ALPHA,ALPHA,ALPHA-TRIFLUORO-ORTHO-TOLYL)-2-THIOHARNSTOFF
1-BENZOYL-3-P-TOLYL-THIOHARNSTOFF
1-BENZOYL-3-PHENYL-2-THIOHARNSTOFF
1-BENZOYL-3-PYRIDIN-2-YLMETHYL-THIOHARNSTOFF
1-(BENZOYLAMIDINO)-2-THIOHARNSTOFF
1-BENZYL-1-BUTYL-3-PHENYL-2-THIOHARNSTOFF
1-BENZYL-1-ETHYL-3-PHENYL-2-THIOHARNSTOFF
1-BENZYL-2-THIOHARNSTOFF
1-BENZYL-3-(2-METHYL-BENZOYL)-THIOHARNSTOFF
1-BENZYL-3-(2-PHENOXYPROPIONYL)-2-THIOHARNSTOFF
1-BENZYL-3-(4-CHLORO-2-(TRIFLUOROMETHYL)PHENYL)-2-THIOHARNSTOFF
1-BENZYL-3-(4-FLUORO-3-NITROPHENYL)-2-THIOHARNSTOFF
1-BENZYL-3-(4-TRIFLUOROMETHYLSULFANYL-PHENYL)-THIOHARNSTOFF
1-BENZYL-3-CINNAMOYL-2-THIOHARNSTOFF
1-BENZYL-3-CYCLOHEXYL-1-ETHYL-2-THIOHARNSTOFF
1-BENZYL-3-FURFURYL-2-THIOHARNSTOFF
1-BENZYL-3-METHYL-2-THIOHARNSTOFF
1-BENZYL-3-PHENYL-2-THIOHARNSTOFF
1-BUTYL-2-THIOHARNSTOFF
1-BUTYL-3-(2-PYRIDYL)-2-THIOHARNSTOFF
1-BUTYL-3-(4-ISOPROPYLPHENYL)-2-THIOHARNSTOFF
1-BUTYL-3-PHENYL-2-THIOHARNSTOFF
1-CINNAMOYL-3-(4-ETHOXYPHENYL)-2-THIOHARNSTOFF
1-CINNAMOYL-3-(4-HYDROXYPHENYL)-2-THIOHARNSTOFF
1-Cyano-3-methylisoTHIOHARNSTOFF, Natriumsalz
1-CYCLODODECYL-3-(4-ETHOXYPHENYL)-2-THIOHARNSTOFF
1-CYCLOHEXYL-1-METHYL-3-PHENYL-2-THIOHARNSTOFF
1-CYCLOHEXYL-3-(2-ETHOXYPHENYL)-2-THIOHARNSTOFF
1-Cyclohexyl-3-(2-morpholinoethyl)-2-THIOHARNSTOFF
1-CYCLOHEXYL-3-(4-ETHOXYPHENYL)-2-THIOHARNSTOFF
1-CYCLOHEXYL-3-PHENYL-2-THIOHARNSTOFF
1-CYCLOPENTYL-3-PHENYL-2-THIOHARNSTOFF
1-DIPHENYLMETHYL-3-(2-PHENETHYL)-2-THIOHARNSTOFF
1-DODECANOYL-2-THIOHARNSTOFF
1-DODECANOYL-3-(1-NAPHTHYL)-2-THIOHARNSTOFF
1-(DODECANOYL)-3-(2-ETHOXYPHENYL)-2-THIOHARNSTOFF
1-DODECANOYL-3-(2-METHOXY-4-NITROPHENYL)-2-THIOHARNSTOFF
1-DODECANOYL-3-(2-METHYL-4-NITROPHENYL)-2-THIOHARNSTOFF
1-DODECANOYL-3-(2-METHYL-5-NITROPHENYL)-2-THIOHARNSTOFF
1-DODECANOYL-3-(2-NAPHTHYL)-2-THIOHARNSTOFF
1-DODECANOYL-3-(2-PYRIMIDINYL)-2-THIOHARNSTOFF
1-DODECANOYL-3-(2-(TRIFLUOROMETHYL)PHENYL)-2-THIOHARNSTOFF
1-DODECANOYL-3-(4-METHOXYPHENYL)-2-THIOHARNSTOFF
1-DODECANOYL-3-(4-(N-METHYLACETAMIDO)PHENYL)-2-THIOHARNSTOFF
1-DODECYL-3-PHENYL-2-THIOHARNSTOFF
1-ETHYL-3-(2-FLUOROPHENYL)-2-THIOHARNSTOFF
1-ETHYL-3-(2-METHOXY-5-METHYLPHENYL)-2-THIOHARNSTOFF
1-ETHYL-3-(3,4-XYLYL)-2-THIOHARNSTOFF
1-ETHYL-3-(4-NITROPHENYL)-2-THIOHARNSTOFF
1-ETHYL-3-(M-TOLYL)-2-THIOHARNSTOFF
1-ETHYL-3-(P-TOLYL)-2-THIOHARNSTOFF
1-ETHYL-3-PHENYL-2-THIOHARNSTOFF
1-(FURAN-2-CARBONYL)-3-(2-TRIFLUOROMETHYL-PHENYL)-THIOHARNSTOFF
1-(FURAN-2-CARBONYL)-3-(5-METHYL-PYRIDIN-2-YL)-THIOHARNSTOFF
1-(FURAN-2-CARBONYL)-3-FURAN-2-YLMETHYL-THIOHARNSTOFF
1-FURAN-2-YLMETHYL-3-(2-METHYL-BENZOYL)-THIOHARNSTOFF
1-FURAN-2-YLMETHYL-3-(4-(1,1,2,2,3,3,3-HEPTAFLUORO-PROPYLSULFANYL)-PH)-THIOHARNSTOFF
1-FURAN-2-YLMETHYL-3-(4-NITRO-BENZOYL)-THIOHARNSTOFF
1-FURFURYL-3-(1-NAPHTHYL)-2-THIOHARNSTOFF
1-FURFURYL-3-PHENYL-2-THIOHARNSTOFF
1-HEXADECANOYL-2-THIOHARNSTOFF
1-HEXADECYL-3-PHENYL-2-THIOHARNSTOFF
1-(HEXAMETHYLENEIMINO)-3-(3-(TRIFLUOROMETHYL)PHENYL)-2-THIOHARNSTOFF
1-ISOBUTYL-3-PHENYL-2-THIOHARNSTOFF
1-ISOPROPYL-3-(1-NAPHTHYL)-2-THIOHARNSTOFF
1-ISOPROPYL-3-(3-(TRIFLUOROMETHYL)PHENYL)-2-THIOHARNSTOFF
1-(ISOPROPYL)-3-(4-METHYL-3-NITROPHENYL)-2-THIOHARNSTOFF
1-ISOPROPYL-3-PHENYL-2-THIOHARNSTOFF
1-METHALLYL-3-METHYL-2-THIOHARNSTOFF
1-METHYL-1-(4-OCTYLOXYPHENYL)-2-THIOHARNSTOFF
1-METHYL-3-(2,4,5-TRICHLOROPHENYL)-2-THIOHARNSTOFF
1-METHYL-3-(2,6-XYLYL)-2-THIOHARNSTOFF
1-METHYL-3-PHENYL-2-THIOHARNSTOFF
1-METHYL-3-PROPYL-2-THIOHARNSTOFF
1-(NAPHTHALENE-1-CARBONYL)-3-M-TOLYL-THIOHARNSTOFF

1-(NAPHTHALENE-1-CARBONYL)-3-PHENYL-THIOHARNSTOFF
1-(NAPHTHALENE-2-CARBONYL)-3-(3-NITRO-PHENYL)-THIOHARNSTOFF
1-(NAPHTHALENE-2-CARBONYL)-3-P-TOLYL-THIOHARNSTOFF
1-OCTADECYL-3-PHENYL-2-THIOHARNSTOFF
1-PHENETHYL-3-PHENYL-2-THIOHARNSTOFF
1-PHENYL-3-(1,2,4)TRIAZOL-4-YL-THIOHARNSTOFF
1-PHENYL-3-(2-(2-PYRIDYL)ETHYL)-2-THIOHARNSTOFF
1-PHENYL-3-(2-(3-PHENYL-THIOUREIDO)-CYCLOHEXYL)-THIOHARNSTOFF
1-PHENYL-3-(2,6-XYLYL)-2-THIOHARNSTOFF
1-PHENYL-3-(2-PYRIDYL)-2-THIOHARNSTOFF
1-Phenyl-3-(2-thiazolyl)-2-THIOHARNSTOFF (2)
1-PHENYL-3-(3-PYRIDYL)-2-THIOHARNSTOFF
1-PHENYL-3-(3-PYRIDYLMETHYL)-2-THIOHARNSTOFF
1-PHENYL-3-(4-(3-PHENYL-THIOUREIDO)-PHENYL)-THIOHARNSTOFF
1-PHENYL-3-(4-TRIFLUOROMETHYL-BENZYL)-THIOHARNSTOFF
1-PHENYL-3-(5-PHENYL-1,2,4-THIADIAZOL-3-YL)-2-THIOHARNSTOFF
1-PHENYL-3-(P-TOLYL)-2-THIOHARNSTOFF
1-PHENYL-3-(P-TOLYLSULFONYL)-2-THIOHARNSTOFF
1-PHENYL-3-PROPYL-2-THIOHARNSTOFF
1-PHENYL-3-TETRADECYL-2-THIOHARNSTOFF
1-PYRIDIN-3-YLMETHYL-3-(2-TRIFLUOROMETHYL-PHENYL)-THIOHARNSTOFF 1-TERT-BUTYL-3-PHENYL-THIOHARNSTOFF
2-(1,1-DIOXO-2,5-DIHYDRO-1H-THIOPHEN-3-YLMETHYL)-ISOTHIOHARNSTOFF, HYDROCHLORIDE
2-(2-Aminoethyl)ISOTHIOHARNSTOFF
2-(2-CYANO-ETHYL)-ISOTHIOHARNSTOFF
2-(3-CARBAMIMIDOYLSULFANYL-ME-2,4,6-TRI-ME-BENZYL)-ISOTHIOHARNSTOFF
2-(3-CARBAMIMIDOYLSULFANYLMETHYL-BENZYL)-ISOTHIOHARNSTOFF
2-(3-CARBAMIMIDOYLSULFANYLMETHYL-BENZYL)-ISOTHIOHARNSTOFF
(2,3-Difluorophenyl)THIOHARNSTOFF
(2,3-DIMETHYL-PHENYL)-THIOHARNSTOFF
2-(3-PHENYL-BUTYL)-ISOTHIOHARNSTOFF
(2,4,6-TRIMETHYL-PHENYL)-THIOHARNSTOFF
2-(4-CARBAMIMIDOYLSULFANYLMETHYL-BENZYL)-ISOTHIOHARNSTOFF
2-(4-CARBAMIMIDOYLSULFANYLMETHYL-BENZYL)-ISOTHIOHARNSTOFF
(2,4-Difluorophenyl)THIOHARNSTOFF
2-(4-HYDROXY-1,1-DIOXO-TETRAHYDRO-THIOPHEN-3-YL)-ISOTHIOHARNSTOFF
2-(4-ME-1,1-DIOXO-4H-THIOPHEN-3-YL)-ISOTHIOHARNSTOFF
2-(4-METHYL-1,1-DIOXO-2,3-DIHYDRO-1H-THIOPHEN-3-YL)-ISOTHIOHARNSTOFF
2-(4-METHYL-1,1-DIOXO-2,5-DIHYDRO-1H-THIOPHEN-3-YL)-ISOTHIOHARNSTOFF
(2,5-Difluorophenyl)THIOHARNSTOFF
2-(6-CARBAMIMIDOYLSULFANYL-ME-NAPHTHALEN-2-YL-ME)-ISOTHIOHARNSTOFF
(2,6-Difluorophenyl)THIOHARNSTOFF
2-BENZOYL-ISOTHIOHARNSTOFF
2-BENZYL-1-(3-(2-MERCAPTO-ETHYL)-3H-BENZOTHIAZOL-2-YLIDENE)-ISOTHIOHARNSTOFF
2-HYDROXY-3-IMINOMETHYL-BENZOIC ACID, COMPOUND WITH THIOHARNSTOFF
(3,4,5-TRIMETHOXY-PHENYL)-THIOHARNSTOFF
3-(4-CHLOROPHENYL)-1,1-DIMETHYL-2-THIOHARNSTOFF
3-(ADAMANTANE-1-CARBONYL)-1,1-DIETHYL-THIOHARNSTOFF
3-ALLYL-1-(3-CHLOROBENZYL)-1-METHYL-2-THIOHARNSTOFF
3-BENZOYL-1,1-DIALLYL-2-THIOHARNSTOFF
3-BENZOYL-1,1-DIMETHYL-THIOHARNSTOFF
3-BENZYL-1,1-DIMETHYL-2-THIOHARNSTOFF
(3-DIMETHYLAMINO-PROPYL)-THIOHARNSTOFF
(3-Fluorophenyl)THIOHARNSTOFF
[3-(Trifluoromethyl)phenyl]THIOHARNSTOFF
(4-ETHOXY-PHENYL)-THIOHARNSTOFF
(4-Flourophenyl)THIOHARNSTOFF
AcetylTHIOHARNSTOFF
Ethylenebis(ISOTHIOHARNSTOFF)
N-(1,1-DIOXIDOTETRAHYDRO-3-THIENYL)-N'-(2-FUROYL)-N-METHYLTHIOHARNSTOFF
N'-(1,3-DIMETHYL-2,4-DIOXO-1,2,3,4-TETRAHYDRO-5-PYRIMIDINYL)N, N-DIMETHYLTHIOHARNSTOFF
N-((1,3-DIOXO-3,4-DIHYDRO-2(1H)-NAPHTHALENYLIDENE)METHYL)-N'-OCTADECYLTHIOHARNSTOFF
N-(1-(4-BR-PHENYL)ETHYLIDENE)-N'-(4-ME-6-OXO-1,6-DIHYDRO-2-PYRIMIDINYL)THIOHARNSTOFF
N-(1-METHYL-2-PHENYLETHYL)THIOHARNSTOFF
N-(1-METHYL-2-PROPENYL)-N'-(4-MORPHOLINYL)THIOHARNSTOFF
N-(2-((1,1,2,2,3,3,3-HEPTAFLUOROPROPYL)THIO)PH)-N'-(2-THIENYLCARBONYL)THIOHARNSTOFF
N-(2-((2,4-DICHLOROBENZYL)SULFONYL)-2-(2,4-DICHLOROPHENYL)VINYL)THIOHARNSTOFF
N-(2,2-DICYANOVINYL)THIOHARNSTOFF
N-(2,2-DIMETHYLPROPANOYL)-N'-(2-NITROPHENYL)THIOHARNSTOFF
N-(2-(2-OXO-1-PYRROLIDINYL)ETHYL)-N'-PHENYLTHIOHARNSTOFF
N-(2-(3,4-DIMETHOXY-PH)ET)-N'-((2,4-DIOXO-2H-CHROMEN-3(4H)-YLIDENE)ME)THIOHARNSTOFF
N-(2,3-DICHLOROPHENYL)-N'-(2-METHYL-3-FUROYL)THIOHARNSTOFF
N-(2,3-DIHYDRO-1,4-BENZODIOXIN-2-YLMETHYL)-N'-(4-METHYLBENZOYL)THIOHARNSTOFF
N-(2,4,6-Trichlorophenyl)THIOHARNSTOFF
N-((2,4,6-TRIOXOTETRAHYDRO-5(2H)-PYRIMIDINYLIDENE)METHYL)THIOHARNSTOFF
N-(2,4-DICHLOROBENZOYL)-N'-(2-PYRIDINYLMETHYL)THIOHARNSTOFF
N-(2,4-DICHLOROBENZOYL)-N'-(TETRAHYDRO-2-FURANYLMETHYL)THIOHARNSTOFF
N-(2,4-DICHLOROPHENYL)-N'-(2,2,2-TRICHLORO-1-(FORMYLAMINO)ETHYL)THIOHARNSTOFF
N-(2,4-DICHLOROPHENYL)-N'-(2-METHYLBENZOYL)THIOHARNSTOFF
N-(2,4-DIFLUOROPHENYL)-N'-(2-METHYLBENZOYL)THIOHARNSTOFF
N-(2,4-DIFLUOROPHENYL)-N'-(3-METHYL-2-FUROYL)THIOHARNSTOFF
N-(2,4-DIMETHYLPHENYL)-N'-(2-METHYL-3-FUROYL)THIOHARNSTOFF
N-(2,4-DIMETHYLPHENYL)-N'-(2-METHYLBENZOYL)THIOHARNSTOFF
N-(2,4-DIMETHYLPHENYL)-N'-(3-FUROYL)THIOHARNSTOFF
N-(2,4-DIMETHYLPHENYL)-N'-(3-METHYL-2-FUROYL)THIOHARNSTOFF
N-(2,4-DIMETHYLPHENYL)-N'-(4-METHYLBENZOYL)THIOHARNSTOFF
N-(2,4-DIMETHYLPHENYL)THIOHARNSTOFF
N-((2,4-DIOXO-1,2,3,4-TETRAHYDRO-3-QUINOLINYL)METHYL)-N'-HEXYLTHIOHARNSTOFF
N-(2,4-DIOXO-1,2,3,4-TETRAHYDRO-5-PYRIMIDINYL)-N'-(4-METHYLBENZOYL)THIOHARNSTOFF
N-((2,4-DIOXO-2H-CHROMEN-3(4H)-YLIDENE)METHYL)-N'-OCTADECYLTHIOHARNSTOFF
N-((2,4-DIOXO-2H-CHROMEN-3(4H)-YLIDENE)METHYL)-N'-PHENYLTHIOHARNSTOFF
N-(2,5-DICHLOROPHENYL)-N'-(2,2,2-TRICHLORO-1-(FORMYLAMINO)ETHYL)THIOHARNSTOFF

N-(2,5-DICHLOROPHENYL)THIOHARNSTOFF
N-(2,6-DI-ME-PH)-N'-((2,4-DIOXO-1,4-DIHYDRO-3(2H)-QUINOLINYLIDENE)ME)THIOHARNSTOFF
N-(2,6-DICHLOROPHENYL)-N'-(3-FUROYL)THIOHARNSTOFF
N-(2,6-DICHLOROPHENYL)-N'-(5-METHYL-2-FUROYL)THIOHARNSTOFF
N-(2-BENZOYL-3-OXO-3-PHENYL-1-PROPENYL)-N'-(2-TERT-BUTYL-6-ETHYLPHENYL)THIOHARNSTOFF
N-(2-(BENZYLSULFINYL)-2-PHENYLVINYL)-N'-(2,4-DIMETHYLPHENYL)THIOHARNSTOFF
N-(2-(BENZYLSULFINYL)-2-PHENYLVINYL)-N'-(2-TERT-BUTYL-6-ETHYLPHENYL)THIOHARNSTOFF
N-(2-(BENZYLSULFINYL)-2-PHENYLVINYL)-N'-PHENYLTHIOHARNSTOFF
N-(2-BROMOBENZOYL)-N'-(2-FURYLMETHYL)THIOHARNSTOFF
N-(2-CHLOROBENZOYL)-N'-(5-(3-(2-FURYL)ACRYLOYL)-4-ME-1,3-THIAZOL-2-YL)THIOHARNSTOFF
N-(2-CHLOROPHENYL)-N'-((6-ME-2,4-DIOXO-2H-PYRAN-3(4H)-YLIDENE)METHYL)THIOHARNSTOFF
N-(2-FLUOROPHENYL)-N'-(2-METHYL-3-FUROYL)THIOHARNSTOFF
N-(2-FLUOROPHENYL)-N'-(3-METHYL-2-FUROYL)THIOHARNSTOFF
N-(2-FLUOROPHENYL)-N'-(5-METHYL-2-FUROYL)THIOHARNSTOFF
N-(2-FUROYL)-N'-(4-METHYLPHENYL)THIOHARNSTOFF
N-(2-FUROYL)-N'-(4-(TRIFLUOROMETHOXY)PHENYL)THIOHARNSTOFF
N-(2-MEO-BENZYLIDENE)-N'-(4-ME-6-OXO-1,6-DIHYDRO-2-PYRIMIDINYL)THIOHARNSTOFF
N-(2-METHYL-3-FUROYL)-N'-(4-(TRIFLUOROMETHYL)PHENYL)THIOHARNSTOFF
N-(2-METHYLBENZOYL)-N'-(2-PYRIDINYL)THIOHARNSTOFF
N-(2-METHYLPHENYL)-N'-(3-ME-5-THIOXO-1,5-DIHYDRO-4H-1,2,4-TRIAZOL-4-YL)THIOHARNSTOFF
N-(3-((1,1,2,2,3,3,3-HEPTAFLUOROPROPYL)THIO)PH)-N'-(2-THIENYLCARBONYL)THIOHARNSTOFF
N-(3,4-DI-CL-BENZOYL)-N'-(5-(3-(2-FURYL)ACRYLOYL)-4-ME-1,3-THIAZOL-2-YL)THIOHARNSTOFF
N-(3,4-DICHLOROPHENYL)-N'-(3-METHYL-2-FUROYL)THIOHARNSTOFF
N-(3,4-DICHLOROPHENYL)-N'-(5-METHYL-2-FUROYL)THIOHARNSTOFF
N-(3,4-DIHYDRO-2H-PYRROL-5-YL)-N-PHENYLTHIOHARNSTOFF
N-(3,4-DIMETHOXYBENZYLIDENE)-N'-(4-ME-6-OXO-1,6-DIHYDRO-2-PYRIMIDINYL)THIOHARNSTOFF
N-(3,4-DIMETHYLPHENYL)THIOHARNSTOFF
N-(3,5-BIS(TRIFLUOROMETHOXY)PHENYL)-N'-(3-FUROYL)THIOHARNSTOFF
N-(3,5-DIBROMO-2-PYRIDINYL)-N'-(2-THIENYLCARBONYL)THIOHARNSTOFF
N-(3,5-DIBROMO-2-PYRIDINYL)-N'-(4-METHYLBENZOYL)THIOHARNSTOFF
N-(3-ACETAMIDOPHENYL)THIOHARNSTOFF
N-(3-BR-BENZOYL)-N'-(5-(3-(3-BR-PHENYL)ACRYLOYL)-4-ME-1,3-THIAZOL-2-YL)THIOHARNSTOFF
N-(3-CARBOXYPHENYL)THIOHARNSTOFF
N-(3-((DIFLUOROMETHYL)THIO)PHENYL)-N'-(2-THIENYLCARBONYL)THIOHARNSTOFF
N-(3-ETHOXY-4-HO-BENZYLIDENE)-N'-(4-ME-6-OXO-1,6-DIHYDRO-2-PYRIMIDINYL)THIOHARNSTOFF
N-(3-FLUOROPHENYL)THIOHARNSTOFF
N-(3-FUROYL)-N'-(4-IODOPHENYL)THIOHARNSTOFF
N-(3-FUROYL)-N'-(4-METHOXYPHENYL)THIOHARNSTOFF
N-(3-FUROYL)-N'-(4-(TRIFLUOROMETHYL)PHENYL)THIOHARNSTOFF
N-(3-HYDROXYPHENYL)THIOHARNSTOFF
N-(3-METHOXYPHENYL)THIOHARNSTOFF
N-(3-METHOXYPHENYL)THIOHARNSTOFF
N-(3-METHYL-2-BUTENOYL)-N'-PHENYLTHIOHARNSTOFF
N-(3-METHYL-2-FUROYL)-N'-(3-(TRIFLUOROMETHYL)PHENYL)THIOHARNSTOFF
N-(3-METHYL-2-FUROYL)-N'-(4-METHYLPHENYL)THIOHARNSTOFF
N-(3-METHYL-2-FUROYL)-N'-(4-(TRIFLUOROMETHYL)PHENYL)THIOHARNSTOFF
N-((3-METHYL-5-OXO-1-PHENYL-1,5-DIHYDRO-4H-PYRAZOL-4-YLIDENE)METHYL)THIOHARNSTOFF
N-(3-METHYL-5-THIOXO-1,5-DIHYDRO-4H-1,2,4-TRIAZOL-4-YL)-N'-PHENYLTHIOHARNSTOFF
N-(3-METHYLPHENYL)-N'-(3-ME-5-THIOXO-1,5-DIHYDRO-4H-1,2,4-TRIAZOL-4-YL)THIOHARNSTOFF
N-(3-METHYLPHENYL)-N'-(4-METHYL-1-PIPERAZINYL)THIOHARNSTOFF
N-(3-(TRIFLUOROMETHYL)PHENYL)-N'-(1,2,2-TRIMETHYLPROPYL)THIOHARNSTOFF
N-(4-(2-THIENYL)-1,3-THIAZOL-2-YL)THIOHARNSTOFF
N-(4-((4-FLUOROBENZYL)THIO)PHENYL)-N'-(4-METHOXYPHENYL)THIOHARNSTOFF
N-(4-(4-MORPHOLINYL)PHENYL)-N'-(2-THIENYLCARBONYL)THIOHARNSTOFF
N-(4-(4-MORPHOLINYL)PHENYL)-N'-(4-NITROBENZOYL)THIOHARNSTOFF
N-(4-ACETAMIDOPHENYL)THIOHARNSTOFF
N-(4-BROMOBENZYLIDENE)-N'-(6-ME-5-OXO-4,5-DIHYDRO-1,2,4-TRIAZIN-3-YL)THIOHARNSTOFF
N-(4-BROMOPHENYL)-N'-(3-FUROYL)THIOHARNSTOFF
N-(4-CARBOXYPHENYL)THIOHARNSTOFF
N-(4-CHLOROBENZYLIDENE)-N'-(4-METHYL-6-OXO-1,6-DIHYDRO-2-PYRIMIDINYL)THIOHARNSTOFF
N-((4-CHLOROPHENOXY)ACETYL)-N'-(2-NITROPHENYL)THIOHARNSTOFF
N-(4-CHLOROPHENYL)-N'-(2,4-DICHLOROBENZOYL)THIOHARNSTOFF
N-(4-CHLOROPHENYL)-N'-(2-FUROYL)THIOHARNSTOFF
N-(4-CHLOROPHENYL)-N'-(2-METHYL-3-FUROYL)THIOHARNSTOFF
N-(4-CHLOROPHENYL)-N'-(3-METHYL-2-FUROYL)THIOHARNSTOFF
N-(4-((DIFLUOROMETHYL)THIO)PHENYL)-N'-(2-THIENYLCARBONYL)THIOHARNSTOFF
N-(4-((DIFLUOROMETHYL)THIO)PHENYL)-N'-(PHENYLACETYL)THIOHARNSTOFF
N-(4-ETHOXYPHENYL)-N'-(4-METHYL-1-PIPERAZINYL)THIOHARNSTOFF
N-(4-ETHOXYPHENYL)THIOHARNSTOFF
N-(4-FLUOROPHENYL)-N'-(2-METHYL-3-FUROYL)THIOHARNSTOFF
N-(4-FLUOROPHENYL)-N'-(3-METHYL-2-FUROYL)THIOHARNSTOFF
N-(4-FLUOROPHENYL)-N'-(4-METHYLBENZOYL)THIOHARNSTOFF
N-(4-FLUOROPHENYL)-N'-(5-METHYL-2-FUROYL)THIOHARNSTOFF
N-(4-FLUOROPHENYL)-N'-(PHENYL(((PHENYLSULFONYL)METHYL)IMINO)METHYL)THIOHARNSTOFF
N-(4-FLUOROPHENYL)THIOHARNSTOFF
N-(4-HYDROXYPHENYL)THIOHARNSTOFF
N-(4-IODOPHENYL)-N'-(2-METHYL-3-FUROYL)THIOHARNSTOFF
N-(4-ME-6-OXO-1,6-DIHYDRO-2-PYRIMIDINYL)-N'-(1-(4-NITRO-PH)ETHYLIDENE)THIOHARNSTOFF
N-(4-METHOXYBENZYLIDENE)-N'-(4-METHYL-6-OXO-1,6-DIHYDRO-2-PYRIMIDINYL)THIOHARNSTOFF
N-(4-METHOXYBENZYLIDENE)-N'-(6-ME-5-OXO-4,5-DIHYDRO-1,2,4-TRIAZIN-3-YL)THIOHARNSTOFF
N-(4-METHOXYPHENYL)-N'-(2-METHYL-3-FUROYL)THIOHARNSTOFF
N-(4-METHOXYPHENYL)-N'-(3-ME-5-THIOXO-1,5-DIHYDRO-4H-1,2,4-TRIAZOL-4-YL)THIOHARNSTOFF
N-(4-METHOXYPHENYL)THIOHARNSTOFF

N-(4-METHYL-5-(3-(2-METHYLPHENYL)ACRYLOYL)-1,3-THIAZOL-2-YL)THIOHARNSTOFF
N-(4-METHYL-5-(3-(3-NITROPHENYL)ACRYLOYL)-1,3-THIAZOL-2-YL)THIOHARNSTOFF
N-(4-METHYL-6-OXO-1,6-DIHYDRO-2-PYRIMIDINYL)-N'-(2-NITROBENZYLIDENE)THIOHARNSTOFF
N-(4-METHYLBENZOYL)-N'-(4-(1-PIPERIDINYL)PHENYL)THIOHARNSTOFF
N-(4-METHYLPHENYL)-N'-(PHENYL(((PHENYLSULFONYL)METHYL)IMINO)METHYL)THIOHARNSTOFF
N-(4-METHYLPHENYL)THIOHARNSTOFF
N-(4-NITROBENZOYL)-N'-(1,3-THIAZOL-2-YL)THIOHARNSTOFF
N-(4-NITROBENZOYL)-N'-(4-NITROPHENYL)THIOHARNSTOFF
N-(4-SULFAMOYL)PHENYL)THIOHARNSTOFF
N-(4-TERT-BUTYLPHENYL)-N'-(2-METHYL-3-FUROYL)THIOHARNSTOFF
N-(4-TERT-BUTYLPHENYL)-N'-(2-METHYLBENZOYL)THIOHARNSTOFF
N-(4-TERT-BUTYLPHENYL)-N'-(2-THIENYLCARBONYL)THIOHARNSTOFF
N-(4-TERT-BUTYLPHENYL)-N'-(3-FUROYL)THIOHARNSTOFF
N-(4-TERT-BUTYLPHENYL)-N'-(3-METHYL-2-FUROYL)THIOHARNSTOFF
N-(5-(2,4-DICHLOROPHENYL)-1,3,4-OXADIAZOL-2-YL)THIOHARNSTOFF
N-(5-(3-(2,4-DICHLOROPHENYL)ACRYLOYL)-4-METHYL-1,3-THIAZOL-2-YL)THIOHARNSTOFF
N-(5-(3-(2-FURYL)ACRYLOYL)-4-METHYL-1,3-THIAZOL-2-YL)-N'-HEXANOYLTHIOHARNSTOFF
N-(5-(3-(2-FURYL)ACRYLOYL)-4-METHYL-1,3-THIAZOL-2-YL)-N'-PALMITOYLTHIOHARNSTOFF
N-(5-(3-(2-FURYL)ACRYLOYL)-4-METHYL-1,3-THIAZOL-2-YL)-N'-PROPIONYLTHIOHARNSTOFF
N-(5-(3-(2-HO-PH)ACRYLOYL)-4-ME-1,3-THIAZOL-2-YL)-N'-(4-NITROBENZOYL)THIOHARNSTOFF
N-(5-(3-(3,4-DIMETHOXYPHENYL)ACRYLOYL)-4-METHYL-1,3-THIAZOL-2-YL)THIOHARNSTOFF
N-(5-(3-(3-BROMOPHENYL)ACRYLOYL)-4-ME-1,3-THIAZOL-2-YL)-N'-ISOBUTYRYLTHIOHARNSTOFF
N-(5-(3-(3-BROMOPHENYL)ACRYLOYL)-4-METHYL-1,3-THIAZOL-2-YL)-N'-PROPIONYLTHIOHARNSTOFF
N-(5-(3-(4-CHLOROPHENYL)ACRYLOYL)-4-METHYL-1,3-THIAZOL-2-YL)THIOHARNSTOFF
N-(5-(3-(4-METHOXYPHENYL)ACRYLOYL)-4-METHYL-1,3-THIAZOL-2-YL)THIOHARNSTOFF
N-(5-(4-METHYLPHENYL)-1,3,4-OXADIAZOL-2-YL)THIOHARNSTOFF
N-(5-BROMO-2-METHOXYBENZYL)-N-(4-ISOPROPYLPHENYL)-N'-PHENYLTHIOHARNSTOFF
N-(5-METHYL-2-FUROYL)-N'-(2-(TRIFLUOROMETHYL)PHENYL)THIOHARNSTOFF
N-(5-(PHENOXYMETHYL)-1,3,4-OXADIAZOL-2-YL)THIOHARNSTOFF
N-(6-(1,3-DIOXO-1,3-DIHYDRO-2H-ISOINDOL-2-YL)HEXANOYL)-N'-(2-MEO-PHENYL)THIOHARNSTOFF
N-((6-METHYL-2,4-DIOXO-2H-PYRAN-3(4H)-YLIDENE)METHYL)-N'-OCTADECYLTHIOHARNSTOFF
N-((6-METHYL-2,4-DIOXO-2H-PYRAN-3(4H)-YLIDENE)METHYL)-N'-PHENYLTHIOHARNSTOFF
N-((6-METHYL-2,4-DIOXO-2H-PYRAN-3(4H)-YLIDENE)METHYL)THIOHARNSTOFF
N-(9-Anthrylmethyl)-N'-benzoyl-N-methyITHIOHARNSTOFF
N-ALLYL-N'-((2,4-DIOXO-1,2,3,4-TETRAHYDRO-3-QUINOLINYL)METHYL)THIOHARNSTOFF
N-ALLYL-N'-((2,4-DIOXO-2H-CHROMEN-3(4H)-YLIDENE)METHYL)THIOHARNSTOFF
N-ALLYL-N'-(4-METHYL-1-PIPERAZINYL)THIOHARNSTOFF
*N*-AllylTHIOHARNSTOFF (2)
N-BENZOYL-N'-(1-NAPHTHYL)THIOHARNSTOFF
N-BENZOYL-N'-(2-NITROPHENYL)THIOHARNSTOFF
N-BENZOYL-N'-(3-METHYLPHENYL)THIOHARNSTOFF
N-BENZOYL-N'-(3-PYRIDINYL)THIOHARNSTOFF
N-BENZOYL-N'-(4-CHLOROPHENYL)THIOHARNSTOFF
N-BENZOYL-N'-(4-METHYL-6-OXO-1,6-DIHYDRO-2-PYRIMIDINYL)THIOHARNSTOFF
N'-Benzoyl-N,N-diethyITHIOHARNSTOFF
N'-Benzoyl-N,N-dihexylTHIOHARNSTOFF
N'-Benzoyl-N,N-diisobutylTHIOHARNSTOFF
N-BENZYL-N'-((1,3-DIOXO-1,3-DIHYDRO-2H-INDEN-2-YLIDENE)METHYL)THIOHARNSTOFF
N-BENZYL-N'-(1,3-THIAZOL-2-YL)THIOHARNSTOFF
N-BENZYL-N'-(2-PYRIDINYL)THIOHARNSTOFF
N-BENZYL-N'-(3-(4-BR-PH)-5-THIOXO-1,5-DIHYDRO-4H-1,2,4-TRIAZOL-4-YL)THIOHARNSTOFF
N-BENZYL-N'-(PHENYL(PHENYLIMINO)METHYL)THIOHARNSTOFF *N*-Boc-THIOHARNSTOFF
N-BUTYL-N'-((2,4-DIOXO-1,4-DIHYDRO-3(2H)-QUINOLINYLIDENE)METHYL)THIOHARNSTOFF
N-CYCLOHEXYL-N'-(1-PHENYLETHYL)THIOHARNSTOFF
N'-CYCLOHEXYL-N,N-DIISOBUTYLTHIOHARNSTOFF
N-Dansyl-N'-ethyITHIOHARNSTOFF
N-DECYL-N'-(2-HYDROXYETHYL)THIOHARNSTOFF
N-ETHYL-N'-(3-METHYL-2-BUTENOYL)-N-PHENYLTHIOHARNSTOFF
*N*-EthyITHIOHARNSTOFF (2)
N-HEXANOYL-N'-(3-PYRIDINYLMETHYL)THIOHARNSTOFF
N-Methallyl-N'-methyITHIOHARNSTOFF
*N*-MethyITHIOHARNSTOFF (2)
N,N-BIS(2-CYANOETHYL)-N'-HEXYLTHIOHARNSTOFF
N,N'-BIS(4-ETHOXYPHENYL)THIOHARNSTOFF
*N,N'*-Di-Boc-S-methylisoTHIOHARNSTOFF
*N,N'*-Di-(*tert*-Butoxycarbonyl)THIOHARNSTOFF
*N,N'*-DibutylTHIOHARNSTOFF (2)
*N,N'*-DiethylTHIOHARNSTOFF
*N,N'*-DimethylTHIOHARNSTOFF
N,N'-DiphenylTHIOHARNSTOFF
*N,N'*-DiphenylTHIOHARNSTOFF
N'-Octadecylfluorescein-5-THIOHARNSTOFF
N-PHENYL-N'-(PHENYL(PHENYLIMINO)METHYL)THIOHARNSTOFF
N-PHENYL-N'-(TETRAHYDRO-2-FURANYLMETHYL)THIOHARNSTOFF
N-PHENYLTHIOHARNSTOFF
*N*-PhenylTHIOHARNSTOFF
N-(TERT-BUTYL)-N'-(3-(TRIFLUOROMETHYL)PHENYL)THIOHARNSTOFF (PHENYL-PHENYLIMINO-METHYL)-THIOHARNSTOFF
PropylTHIOHARNSTOFF
*S*-[(2-Guanidino-4-thiazoyl)methyl] ISOTHIOHARNSTOFF
S-BenzylisoTHIOHARNSTOFF
S-EthylisoTHIOHARNSTOFF
S-IsopropylisoTHIOHARNSTOFF
S-METHYL-N-(4-TOLUENESULFONYL)ISOTHIOHARNSTOFF
*S*-MethylisoTHIOHARNSTOFF
TetramethyITHIOHARNSTOFF
THIOHARNSTOFF
TOLUENE-4-SULFONSÄURE, 2-(2-(1-OXY-PYRIDIN-2-YL)-ETHYL)-ISOTHIOHARNSTOFF
1,3-Bis(benzyloxycarbonyl)-2-methyl-2-thiopseudoharnstoff
1,3-Bis(*tert*-butoxycarbonyl)-2-methyl-2-thiopseudoharnstoff
2-Benzyl-2-thiopseudoharnstoff
2-Ethyl-2-thiopseudoharnstoff
2-Imidazolidinethione
2-Imino-4-thiobiuret
(Amidinothio)essigsäure
Formamidinsulfinsäure
*S*-(2-Aminoethyl)isothiouroniumbromid

Als Kupferverbindungen kommen vor allem Kupfersalze und -Komplexe in Frage, wie etwa Kupferbenzoat, Kupferdi(methacrylat), Kupferacetylacetonat oder Kupfernaphthenat in Frage.

Als Monomere ohne eine Säurefunktion kommen neben den Vinyl- und Vinylcyclopropanmonomeren vor allem Acrylate und Methacrylate in Frage.
Monofunktionelle oder vernetzende' (Meth)acrylate können dabei sein: Monofunktionelle oder polyfunktionelle (Meth)acrylate, die alleine oder in Mischungen eingesetzt werden können. Als Beispiele für diese Verbindungen kommen Methylmethacrylat, Isobutylmethacrylat, Cyclohexylmethacrylat, Triethylenglycoldimethacrylat, Diethylenglycoldimethacrylat, Tetraethylenglycoldimethacrylat, Ethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, ethoxyliertes Bisphenol-A-dimethacrylat, aber auch Bis-GMA (2,2-bis-4-(3-methacryloxy-2-hydroxypropyl)-phenylpropan) sowie die Reaktionsprodukte aus Isocyanaten, insbesondere Di- und/oder Triisocyanaten und OH-gruppenhaltigen Methacrylaten, und die entsprechenden Acrylate aller obigen Verbindungen in Frage. Beispiele für Reaktionsprodukte von Isocyanaten sind die Umsetzungsprodukte von 1 Mol Hexamethylendiisocyanat mit 2 Mol 2-Hydroxyethylmethacrylat, von 1 Mol (Tri(6-isocyanatohexyl)biuret mit 3 Mol Hydroxyethylmethacrylat und von 1 Mol Trimethylhexamethylendiisocyanat mit 2 Mol Hydroxyethylmethacrylat, die auch als Urethandimethacrylate bezeichnet werden. Geeignete Monomere sind jeweils die Monomere selbst, daraus hergestellte polymerisierbare Präpolymere sowie Mischungen von diesen.
¹ Vernetzende Meth-/acrylate sind naturgemäß Verbindungen mit 2 oder mehr Methacrylatgruppen im Momomeren

Bevorzugte Vernetzermonomere sind z.B. 2,2-Bis-4-(3-methacryloxy-2-hydroxypropyl)-phenylpropan) (Bis-GMA), d.h. das Umsetzungsprodukt von Glycidylmethacrylat und Bisphenol-A (OH-gruppenhaltig), und 7,7,9-Trimethyl-4,13-dioxo-3,14-dioxa-5,12-diazahexadecan-1,16-diyldimethacrylat (UDMA), d.h. das Urethandimethacrylat aus 2 Mol 2-Hydroxyethylmethacrylat (HEMA) und 1 Mol 2-2,4-Trimethylhexamethylendiisocyanat (urethangruppenhaltig). Darüber hinaus sind Umsetzungsprodukte von Glycidylmethacrylat mit anderen Bisphenolen, wie z.B. Bisphenol-B (2,2'-Bis-(4-hydroxyphenyl)-butan), Bisphenol-F (2,2'-Methylendiphenol) oder 4,4'-Dihydroxydiphenyl, sowie Umsetzungsprodukte von 2 Mol HEMA oder 2-Hydroxypropyl(meth)-acrylat mit, insbesondere 1 Mol, bekannter Diisocyanate, wie z.B. Hexamethylendiisocyanat, m-Xylylendiisocyanat oder Toluylendiisocyanat, als Vernetzermonomere bevorzugt.

### Versuchsbeschreibunclen / Beispiele:

Die beiden Komponenten, nachfolgend als Basis- und Katalysator-Komponente (B bzw. C) bezeichnet, wurden nach einer allgemeinen Herstellvorschrift gefertigt. Dazu wurden die Monomere zunächst zu einer homogenen Mischung vermengt. Anschließend wurden die Initiatorkomponenten zugegeben und durch intensives Rühren mit dem Magnetrührer gelöst.

An den nachfolgenden Zusammensetzungen wurden die besonderen Eigenschaften des erfindungsgemäßen 2K-Initiatorsystems näher untersucht. Die Komponenten B1 in Kombination mit C1 und C2 stellen die Referenzsysteme dar.

### Basis-Komponenten

| | | |
|---|---|---|
| B1 | Bis-GMA | 20,580 |
| | TEGDMA | 8,820 |
| | DBPO | 0,600 |
| | | |
| B2 | Bis-GMA | 20,790 |
| | TEGDMA | 8,910 |
| | CHP | 0,300 |
| | | |
| B10 | Bis-GMA | 19,347 |
| | TEGDMA | 8,291 |
| | Acrylsäure | 1,455 |
| | CHP | 0,907 (Cumenhydroperoxid) |
| | | |
| B122 | Bis-GMA | 17,64 |
| | TEGDMA | 7,56 |
| | CHP | 0,300 (1,00%) |
| | 4-Meta | 4,500 (15,00%) |
| | | |
| B123 | Bis-GMA | 20,37 |
| | TEGDMA | 8,73 |
| | CHP | 0,300 (1,00%) |
| | Phosphorsäureester | 0,600 (2,00%) |
| | | |
| B124 | Bis-GMA | 19,74 |
| | TEGDMA | 8,46 |
| | CHP | 0,300 (1,00%) |
| | Phosphorsäureester | 1,500 (5,00%) |

### Katalysator-Komponenten

| | | |
|---|---|---|
| C1 | Bis-GMA | 20,895 |
| | TEGDMA | 8,955 |
| | DMPT | 0,150 |
| | | |
| C2 | Bis-GMA | 20,895 |
| | TEGDMA | 8,955 |
| | DHEPT | 0,150 |
| | | |
| C3 | Bis-GMA | 20,790 |
| | TEGDMA | 8,910 |
| | ATH | 0,300 |
| | | |
| C3.1 | Bis-GMA | 20,51 |
| | TEGDMA | 8,79 |
| | ATH | 0,900 |
| | | |
| C3.4 | Bis-GMA | 20,769 |
| | TEGDMA | 8,901 |
| | ATH | 0,300 |
| | Cu(acac)₂ | 0,030 |
| | | |
| C3.5 | Bis-GMA | 20,348 |
| | TEGDMA | 8,721 |
| | ATH | 0,901 |
| | Cu(acac)₂ | 0,029 |
| | | |
| C15 | Bis-GMA | 20,348 |
| | TEGDMA | 8,721 |
| | ATH | 0,901 |
| | Kupfer(II)-naphthenat | 0,029 |
| | | |
| C121 | Bis-GMA | 20,782 |
| | TEGDMA | 8,906 |
| | ATH | 0,300 (1,00%) |
| | Cu(ac)₂ | 0,012 (0,04%) |
| | | |
| C122 | Bis-GMA | 17,632 |
| | TEGDMA | 7,556 |
| | 4-Meta | 4,5 (15,00%) |
| | ATH | 0,300 (1,00%) |
| | Cu(ac)2 | 0,012 (0,04%) |
| | | |
| C123 | Bis-GMA | 20,362 |
| | TEGDMA | 8,726 |
| | Sipomer PAM100 | 0,6 (2,00%) |
| | ATH | 0,300 (1,00%) |
| | Cu(ac)2 | 0,012 (0,04%) |
| | | |
| C124 | Bis-GMA | 19,732 |
| | TEGDMA | 8,456 |
| | Sipomer PAM100 | 1,5 (5,00%) |
| | ATH | 0,300 (1,00%) |
| | Cu(ac)2 | 0,012 (0,04%) |

Durch das Einbringen gebräuchlicher Füllstoffe in die aktivierten Monomerharzmischungen kann der Fachmann Komposite für unterschiedliche dentale Anwendungen herstellen. Geeignete Füllstoffe sind insbesondere Quarz- und Glaskeramikpulver, Aluminiumoxide und/oder Siliciumoxide. Besonders bevorzugte Füllstoffe sind Glaspulver, z. B. Bariumglas-, Bariumsilikatglas-, Lithium- oder Aluminium-Silikatglas-Pulver und feinstteilige Kieselsäuren, wie pyrogene oder gefällte Kieselsäuren.

### Prüfung der Gelzeiten

Zur Bestimmung der Verarbeitungszeiten wurden beide Komponenten im Verhältnis 1:1 intensiv vermischt. Die Zeit bis zum Auftreten des Gelpunktes wird als Gelzeit bezeichnet und kann zur Einstellung der maximalen Verarbeitungszeit betrachtet werden. Die sensorische Wahrnehmung unterliegt allerdings einer individuellen Schwankungsbreite, die auch vom Abbindeverhalten des Systems abhängig ist.

Als zusätzliche Methode zur Erfassung der Initiierungscharakteristik wurde die Wärmetönung calorimetrisch registriert. Der beginnende Temperatursanstieg wurde als relevanter Messpunkt bestimmt.

Die Messungen zeigten in Abhängigkeit vom Kupfergehalt der Zubereitungen eine zuverlässig einstellbare Verarbeitungszeit. Die Variationsbreite der Aktivität des Initiatorsystems reicht dabei von extrem kurzen Inhibierungszeiten < 10 s bei größeren Kupfergehalten (um 0,1 %) bis hin zur sehr langen Inhibierungszeiten oder sogar zum Ausbleiben der Polymerisation bei fehlender Kupferverbindung.

### Prüfung der Lagerstabilität

Die Einzelkomponenten wurden bei einer Temperatur von 50 °C dauerhaft gelagert. Die Veränderung der Verarbeitungszeiten wurde über einen Zeitraum von maximal 5 Monaten dokumentiert. Dabei wurden die Proben mit den ebenfalls bei 50 °C bzw. bei Raumtemperatur (RT) gelagerten zweiten Komponenten getestet.
Während die Basis-Komponente des herkömmlichen Dibenzoylperoxid-Amin-Initiatorsystems bereits nach 10 Tagen spontan aushärtete, waren die Komponenten des erfindungsgemäßen

Systems auch nach 5 Monaten noch gebrauchsfähig. Die Aushärtung erfolgte auch in Gegenwart von Adhäsiv-Monomeren unter aciden Bedingungen zuverlässig.
In den Versuchen zeigt sich deutlich der starke katalytische Einfluss des Kupfergehaltes auf die Aktivität des Initiatorsystems. Durch den Anteil einer löslichen Kupferverbindung konnten nachweisbar deutlich kürzere Verarbeitungszeiten eingestellt werden bzw. die Konzentration der Redoxpartner reduziert werden. Beide Möglichkeiten sind besonders vorteilhaft für den Einsatz in Dentalmaterialien bzw. Kunststoffen für den medizinischen Gebrauch, da hier sehr kurze Verarbeitungszeiten und ein möglichst geringes toxikologisches Potential gefordert sind.

### Referenzsystem: Dibenzoylperoxid-Amin

| Referenz - Lagerung der Peroxid-Komponente bei 50 °C | | | |
|---|---|---|---|
| B1 | 1 % DBPO | 0 Tage | 50 s |
| | | 5 Tage | 40 s |
| | | 10 Tage | **polymerisiert** |

| Referenz - Lagerung der Amin-Komponente bei 50 °C | | | |
|---|---|---|---|
| | | | B1 |
| | | | 1 % DBPO |
| C1 | 0,5 % DMPT | 0 Monate | 50 s |
| | | 3 Monate | 95 s |
| | | 5 Monate | - |
| C2 | 0,5 % DHEPT | 0 Monate | 380 s |
| | | 3 Monate | - |
| | | 5 Monate | 420 s |

### System: Cumenhydroperoxid-Acetylthioharnstoff-Derivat

| Lagerung beider Komponenten mit Kupferzugabe bei 50 °C | | | |
|---|---|---|---|
| | | | B2 |
| | | | 1 % CHP |
| C121 | 1 % ATH | 0 Monate | 45 s |
| | 0,04 % Cu(acac)₂ | 1 Monate | - |
| | | 3 Monate | 235 s |
| C3.5 | 1 % ATH | 0 Monate | 35 s |
| | 0,1 % Cu(acac)₂ | 1 Monate | 70 s |
| | | 3 Monate | 345 s |
| C3.4 | 3 % ATH | 0 Monate | 40 s |
| | 0,1 % Cu(acac)₂ | 1 Monate | 80 s |
| | | 3 Monate | 200 s |

| Lagerung der ATH-Komponente mit Adhäsiv-Monomer und Kupferzugabe bei 50 °C | | | |
|---|---|---|---|
| | | | B2 |
| | | | 1 % CHP |
| C121 | 1 % ATH, 0,04 % | 0 Monate | 45 s |
| | Cu(acac)₂ | 3 Monate | 85 s |
| C122 | 1 % ATH, 0,04 % | 0 Monate | 110 s |
| | Cu(acac)₂ | 3 Monate | 160 s |
| | 15 % 4-META | | |
| C123 | 1 % ATH, 0,04 % | 0 Monate | 50 s |
| | Cu(acac)₂ | 3 Monate | 70 s |
| | 2 % Phosphorsäureester | | |
| C124 | 1 % ATH, 0,04 % | 0 Monate | 50 s |
| | Cu(acac)₂ | 3 Monate | 70 s |
| | 5 % Phosphorsäureester | | |

### Abhängigkeit der Inhibierungszeit vom Kupfergehalt

| Einstellung der Verarbeitungszeit am Kupfergehalt | | | |
|---|---|---|---|
| | | | B2 |
| | | | 1 % CHP |
| C3 | 1 % ATH | 0,00 % Cu(acac)₂ | keine Polymerisation |
| | | 0,01 % Cu(acac)₂ | 135 s |
| | | 0,02 % Cu(acac)₂ | 75 s |
| C121 | | 0,04 % Cu(acac)₂ | 45 s |
| | | 0,06 % Cu(acac)₂ | 40 s |
| C3.5 | | 0,10 % Cu(acac)₂ | 35 s |

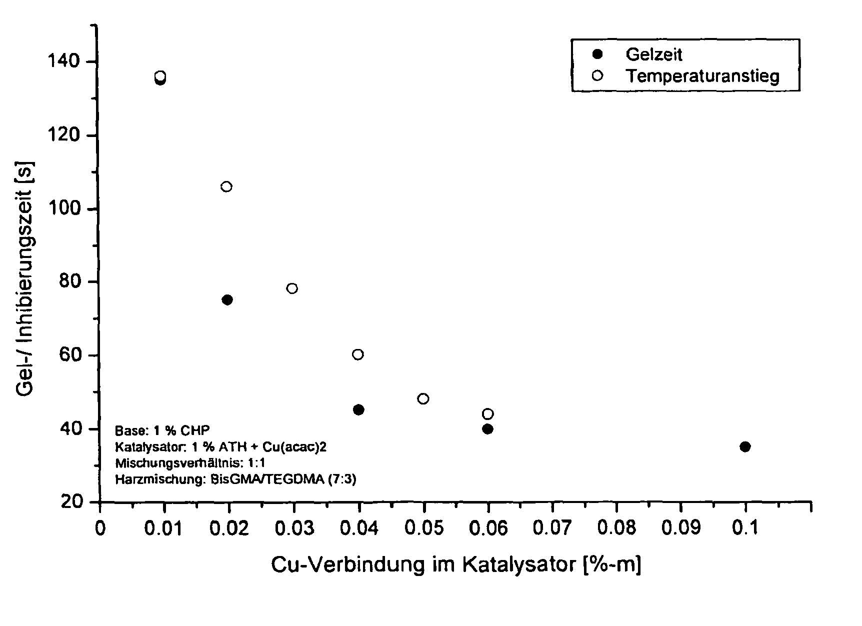

| Wirksamkeit verschiedener Kupfersalze | | | |
|---|---|---|---|
| | | | B2 |
| | | | 1 % CHP |
| C3.5 | 1 % ATH | 0,10 % Kupfer(II)acetonylacetonat | 35 s |
| C15 | | 0,10 % Kupfer(II)naphthenat | 55 s |
| | | | |

### Färbung der Polymere durch den Kupfergehalt

Die Farbe der polymerisierten (Meth-)acrylate wird vom Gehalt an den naturgemäß gefärbten Kupferionen beeinflusst. Der Zusammenhang zwischen der Eigenfärbung der Polymerisate und des Anteils der Kupferverbindung wurde durch Farbmessungen an Prüfkörpern ermittelt. Von besonderer Bedeutung war dabei die Farbkonstanz.
Zur Bestimmung wurden Prüfkörper von 1 mm Dicke hergestellt und nach einer Lagerung von 48 Stunden gemessen. Es wurden die CIE-Lab-Werte der transparenten Polymerschichten vor einem weißen Hintergrund registriert. Die Farbstabilität wurde nach 7 Tagen bei einer Temperatur von 40 °C überprüft. Dabei konnte keine Farbänderung der Schichten beobachtet werden. Auch nach längerer Lagerung erfolgte keine erkennbare Farbänderung, weshalb das erfindungsgemäße Initiatorsystem mit einer Kupferverbindung auch die ästhetischen Anforderungen, die an einen Dentalwerkstoff gestellt werden, voll erfüllt.

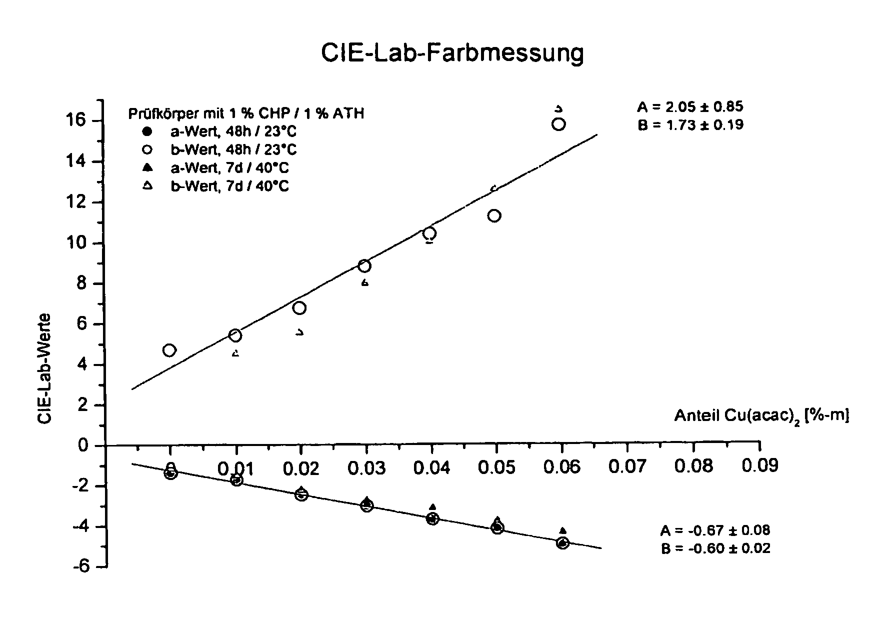

## Patentansprüche

1. Aminfreie Dentalzusammensetzung enthaltend:
(a) mindestens ein polymerisierbares Monomer ohne eine Säurefunktion, wobei die polymerisierbare Gruppe eine Struktureinheit der Acrylate, Methacrylate, einer Vinylgruppe oder eines Vinylcyclopropans enthält;
(b) eine Hydroperoxid-Verbindung mit einer oder mehreren Hydroperoxidgruppen, die an einen teritären Kohlenstoff gebunden sind;
(c) ein Thioharnstoff-Derivat;
(d) eine in der Zubereitung lösliche Kupfer-Verbindung;
(e) eine oder mehrere Adhäsiv-Monomere, die mindestens eine acide Funktion enthalten, ausgewählt aus den Carbonsäuren, Säureanhydriden, Sulfonsäuren, Sulfinsäuren, Phosphorsäureestern, Phosphonsäureestern, Phosphonaten oder Phosphinaten; Phosphonsäuren, Phosphinsäuren, Schwefelsäureester, Borsäureester und Boronsäuren,
(f) mindestens einen Stabilisator;
(g) optional einen Photoinitiator, einen verteilten Füllstoff, ein Lösungsmittel oder Kombinationen davon,
wobei die acide Verbindung (e) in einem Anteil von mindestens 1 Gew.-% enthalten ist.

2. Dentalzusammensetzung nach Anspruch 1, die eine Kupfer-Verbindung in einem Anteil von mindestens 0,0001, höchstens aber 5 Gew.-%, enthält.

3. Dentalzusammensetzung nach Anspruch 2, die eine Kupfer-Verbindung in einem Anteil von mindestens 0,01, höchstens aber 5 Gew.-%, enthält.

4. Dentalzusammensetzung nach einem der vorstehenden Ansprüche, aufgeteilt in zwei Komponenten, wobei eine Zusammensetzung des zweikomponentigen Systems das Hydroperoxid enthält und die zweite Zubereitung das Thioharnstoff-Derivat und mindestens eine Kupfer-Verbindung beinhalten.

5. Dentalzusammensetzung nach einem der vorstehenden Ansprüche, wobei das 2-Komponenten-System eine Kombination von Zubereitungen unterschiedlicher und gleicher Konsistenzen aus den Gruppen: Flüssigkeit/-Flüssigkeit, Paste/Paste, Paste/Flüssigkeit und Pulver/Flüssigkeit darstellt.

6. Dentalzusammensetzung nach Anspruch 4, wobei das 2-Komponenten-System aus einer ersten Paste mit der Zusammensetzung (a), (b), (e), (f) und einem fein verteilten Füllstoff besteht und einer zweiten Paste bestehend aus (a), (c), (d), (e), (f) und einem fein verteilten Füllstoff.

7. Dentalzusammensetzung nach Anspruch 4, wobei das 2-Komponenten-System in mindestens einer Komponente einen Photoinitiator als Bestandteil (g) enthält und nach dem Mischen ein dualhärtendes System bildet.

8. Dentalzusammensetzung nach einem der vorstehenden Ansprüche, wobei die Zubereitung ein Füllungsmaterial, ein Zement, ein Fissurenversiegeler, ein Prothesenmaterial, ein Primer oder ein Adhäsiv oder ein selbstadhäsives Komposit sein kann.

9. Dentalzusammensetzung nach Anspruch 4, wobei das 2-Komponenten-System aus einer ersten Flüssigkeit mit der Zusammensetzung (a), (b), (e), (f) und mindestens einem flüchtigen Lösungsmittel besteht und einer zweiten Flüssigkeit bestehend aus (a), (c), (d), (e), (f) und mindestens einem Lösungsmittel.

10. Dentalzusammensetzung nach den Ansprüchen 4 bis 9, wobei jede der beiden Komponenten in einer Spritze als Packmittel bereitgestellt wird.

11. Dentalzusammensetzung nach den Ansprüchen 4 bis 9, wobei beide Komponenten in einer Doppelkammer-Spritze als Packmittel bereitgestellt werden.

12. Dentalzusammensetzung nach Anspruch 9, wobei die Komponenten der Doppelkammer-Spritze beim Austragen gegebenenfalls durch einen aufgesetzten Statikmischer homogen angemischt werden können.

13. Dentalzusammensetzung nach Anspruch 9, wobei jede der beiden Komponenten in einer Flasche als Packmittel bereitgestellt wird.

14. Dentalzusammensetzung nach den Ansprüchen 4 bis 9, wobei jede der beiden Komponenten in einem unterschiedlichen Packmittel (z.B. Spritze und Flasche) bereitgestellt wird.

15. Dentalzusammensetzung nach den Ansprüchen 4 bis 9, wobei beide Komponenten in einem Doppelkammer-Dispenser als Packmittel getrennt vorliegen und während des Austragens vermischt werden.

16. Dentalzusammensetzung nach Anspruch 4, wobei das Material in Form von Pulver und Flüssigkeit bereitgestellt wird und das Pulver die Bestandteile (c), (d) enthält, während in der Flüssigkeit die Bestandteile (a), (b), (f) und gegebenenfalls (e) enthalten sind.

17. Dentalzusammensetzung nach den Ansprüchen 6 und 16 enthaltend einen Füllstoff, der bestehen kann aus anorganischen Metalloxiden, -nitriden, -salzen, Silikatglas, Aluminosilikatglas, Aluminoborosilikatglas, Fluoroaluminosilikatglas, Quarz, kolloidales Siliziumdioxid, pyrogenes Siliziumdioxid, gefälltes Siliziumdioxid, polymeres Siliziumdioxid, Zirkonoxid-Siliciumdioxid, polymere Füller, polymerisierte Komposit-Füller mit enthaltenen anorganischen Partikeln und Kombinationen davon.

18. Dentalzusammensetzung nach Anspruch 17 enthaltend Füllstoffe aus der Gruppe bestehend aus Metalloxiden, -nitriden, -salzen mindestens eines Elements, ausgewählt aus der Reihe Sr, Y, Zr, Ba, La, Hf, Zn, Bi, W, Lanthaniden oder Kombinationen davon, sowie Silikatglas, Aluminosilikatglas, Aluminoborosilikatglas und Fluoroalumino-silikatglas.

19. Dentalzusammensetzung nach einem der vorstehenden Ansprüche, wobei als Lösungsmittel Wasser, Methanol, Ethanol, Propanol, Isopropanol, Azeton, Methylethylketon, Ethylenglykol, Glyzerin und Kombinationen davon enthalten sein können.

20. Dentalzusammensetzung nach einem der vorstehenden Ansprüche, die eine acide, polymerisierbare Verbindung mit mindestens einer ethylenisch ungesättigten Gruppe enthalten, ausgewählt aus der Gruppe der Acrylate, Methacrylate oder Vinyl-Verbindungen.

21. Dentalzusammensetzung nach einem der vorstehenden Ansprüche, enthaltend mindestens eine Hydroperoxid-Verbindung ausgewählt unter den Substanzen t-Butylhydroperoxid, t-Amylhydroperoxid, iso-Propylbenzenhydroperoxid, p-Diisopropylbenzenhydroperoxid, 5-Phenyl-4-pentenylhydroperoxid, Pinanhydroperoxid, 1,1,3,3-Tetramethylbutylhydroperoxid und Kombinationen davon.

22. Verwendung einer Dentalzusammensetzung nach einem der vorstehenden Ansprüche 5-22 zur Herstellung einer restorativ, orthodontisch oder endodontisch nutzbaren Zubereitung.

23. Dentalzusammensetzung nach einem der vorstehenden Ansprüche 5-22, die eine Abbindezeit nach dem Anmischen von weniger als 15 Minuten erlaubt.

24. Dentalzusammensetzung nach einem der vorstehenden Ansprüche 5-23, die eine Abbindezeit nach dem Anmischen von weniger als 5 Minuten erlaubt.

25. Dentalzusammensetzung nach einem der vorstehenden Ansprüche 5-24, die eine Abbindezeit nach dem Anmischen von weniger als 15 Minuten erlaubt, nachdem beide Komponenten wenigstens 2 Monate bei 50°C gelagert wurden.

26. Dentalzusammensetzung nach einem der vorstehenden Ansprüche 5-25, die eine Abbindezeit nach dem Anmischen von weniger als 15 Minuten erlaubt, nachdem beide Komponenten wenigstens 3 Monate bei 37°C gelagert wurden.

## Claims

1. An amine-free dental composition, containing:
(a) at least one polymerisable monomer with no acid function, whereby the polymerisable group contains a structural unit of the acrylates, methacrylates, of a vinyl group or of a vinylcyclopropane;
(b) a hydroperoxide compound with one or more hydroperoxide groups bound to a tertiary carbon;
(c) a thiourea derivative;
(d) a copper compound that is soluble in the preparation;
(e) one or more adhesive monomers that contain at least one acid function selected from the carbonic acids, acid anhydrides, sulfonic acids, sulfinic acids, phosphoric acid esters, phosphonic acid esters, phosphonates or phosphinates; phosphonic acids, phosphinic acids, sulfuric acid esters, boric acid esters, and boric acids;
(f) at least one stabiliser;
(g) optionally, one photoinitiator, a distributed filling agent, one solvent or combinations thereof;
whereby the acid compound (e) is contained therein accounting for a fraction of at least 1 % by weight.

2. A dental composition according to claim 1, containing a copper compound accounting for a fraction of at least 0.0001, but at most 5 % by weight.

3. A dental composition according to claim 2, containing a copper compound accounting for a fraction of at least 0.01, but at most 5 % by weight.

4. A dental composition according to any one of the preceding claims, subdivided into two components, whereby one composition of the two-component system contains the hydroperoxide and the second preparation contains the thiourea derivative and at least one copper compound.

5. A dental composition according to any one of the preceding claims, whereby the 2-component system is a combination of preparations of different and same consistencies from the groups: liquid/liquid, paste/paste, paste/liquid, and powder/liquid.

6. A dental composition according to claim 4, whereby the 2-component system consists of a first paste of the composition, (a), (b), (e), (f), and a finely distributed filling agent, and a second paste consisting of (a), (c), (d), (e), (f) and a finely distributed filling agent.

7. A dental composition according to claim 4, whereby the 2-component system contains a photoinitiator as ingredient (g) in at least one component and forms a dual-curing system after being mixed.

8. A dental composition according to any one of the preceding claims, whereby the preparation can be a filling material, a cement, a fissure sealant, a prosthetic material, a primer or an adhesive or a self-adhesive composite.

9. A dental composition according to claim 4, whereby the 2-component system consists of a first liquid of the composition, (a), (b), (e), (f), and at least one volatile solvent, and a second liquid consisting of (a), (c), (d), (e), (f), and at least one solvent.

10. A dental composition according to claims 4 to 9, whereby each of the two components is provided in a syringe as packaging means.

11. A dental composition according to claims 4 to 9, whereby both components are provided in a double-chamber syringe as packaging means.

12. A dental composition according to claim 9, whereby the components of the double-chamber syringe, when they are being dispensed, can be mixed homogeneously, if applicable, by a static mixer that is attached to the syringe.

13. A dental composition according to claim 9, whereby each of the two components is provided in a vial as packaging means.

14. A dental composition according to claims 4 to 9, whereby each of the two components is provided in a different packaging means (e.g. syringe and vial).

15. A dental composition according to claims 4 to 9, whereby both components are present separately in one double-chamber dispenser as packaging means, and are mixed while being dispensed.

16. A dental composition according to claim 4, whereby the material is provided in the form of powder and liquid, and the powder contains ingredients (c), (d), whereas the liquid contains ingredients (a), (b), (f) and, if applicable, (e).

17. A dental composition according to claims 6 and 16, containing a filling agent that can consist of inorganic metal oxides, inorganic metal nitrides, inorganic metal salts, silica glass, aluminosilica glass, aluminoborosilica glass, fluoroaluminosilica glass, quartz, colloidal silicon dioxide, pyrogenic silicon dioxide, precipitated silicon dioxide, polymeric silicon dioxide, zirconium oxide-silicon dioxide, polymeric filling agents, polymerised composite filling agents containing inorganic particles, and combinations thereof.

18. A dental composition according to claim 17, containing filling agents from the group consisting of metal oxides, metal nitrides, metal salts of at least one element selected from the series Sr, Y, Zr, Ba, La, Hf, Zn, Bi, W, lanthanides or combinations thereof, as well as silica glass, aluminosilica glass, aluminoborosilica glass, and fluoroaluminosilica glass.

19. A dental composition according to any one of the preceding claims, whereby water, methanol, ethanol, propanol, isopropanol, acetone, methylethylketone, ethylene glycol, glycerol, and combinations thereof can be contained therein as solvent(s).

20. A dental composition according to any one of the preceding claims, containing an acidic, polymerisable compound with at least one ethylenic unsaturated group selected from the group of the acrylates, methacrylates or vinyl compounds.

21. A dental composition according to any one of the preceding claims, containing at least one hydroperoxide compound selected from the substances, t-butyl hydroperoxide, t-amyl hydroperoxide, iso-propylbenzene hydroperoxide, p-diisopropylbenzene hydroperoxide, 5-phenyl-4-pentenyl hydroperoxide, pinane hydroperoxide, 1,1,3,3-tetramethylbutyl hydroperoxide, and combinations thereof.

22. Use of a dental composition according to any one of the preceding claims 5-22 for producing a preparation that can be used in restorative, orthodontic or endodontic applications.

23. A dental composition according to any one of the preceding claims 5-22 that allows for a setting time after mixing of less than 15 minutes.

24. A dental composition according to any one of the preceding claims 5-23 that allows for a setting time after mixing of less than 5 minutes.

25. A dental composition according to any one of the preceding claims 5-24 that allows for a setting time after mixing of less than 15 minutes after storage of both components for at least 2 months at 50°C.

26. A dental composition according to any one of the preceding claims 5-25 that allows for a setting time after mixing of less than 15 minutes after storage of both components for at least 3 months at 37°C.

## Revendications

1. Composition dentaire exempte d'amine contenant :
(a) au moins un monomère polymérisable sans fonction acide, dans laquelle le groupe polymérisable contient un motif structurel des acrylates, méthacrylates, d'un groupe vinyle ou d'un vinylcyclopropane ;
(b) un composé d'hydroperoxyde avec un ou plusieurs groupes hydroperoxyde qui sont liés à un carbone tertiaire ;
(c) un dérivé de thiourée ;
(d) un composé de cuivre soluble dans la préparation ;
(e) un ou plusieurs monomères adhésifs qui contiennent au moins une fonction acide, sélectionnés parmi les acides carboxyliques, anhydrides acides, acides sulfoniques, acides sulfiniques, esters d'acide phosphorique, esters d'acide phosphonique, phosphonates ou phosphinates ; acides phosphoniques, acides phosphiniques, ester d'acide sulfurique, ester d'acide borique et acides boroniques,
(f) au moins un stabilisateur ;
(g) en option, un photoinitiateur, une charge répartie, un solvant ou des combinaisons de ceux-ci,
dans laquelle le composé acide (e) est contenu dans une proportion d'au moins 1 % en poids.

2. Composition dentaire selon la revendication 1, qui contient un composé de cuivre dans une proportion d'au moins 0,0001, mais de 5 % en poids maximum.

3. Composition dentaire selon la revendication 2, qui contient un composé de cuivre dans une proportion d'au moins 0,01, mais de 5 % en poids maximum.

4. Composition dentaire selon l'une quelconque des revendications précédentes, divisée en deux composants, dans laquelle une composition du système bicomposant contient l'hydroperoxyde et la seconde préparation contient le dérivé de thiourée et au moins un composé de cuivre.

5. Composition dentaire selon l'une quelconque des revendications précédentes, dans laquelle le système bicomposant représente une combinaison de préparations de différentes consistances et de consistances identiques provenant des groupes : liquide/liquide, pâte/pâte, pâte/liquide et poudre/liquide.

6. Composition dentaire selon la revendication 4, dans laquelle le système bicomposant se compose d'une première pâte avec la composition (a), (b), (e), (f) et une charge finement répartie et d'une seconde pâte constituée de (a), (c), (d), (e) et (f) et d'une charge finement répartie.

7. Composition dentaire selon la revendication 4, dans laquelle le système bicomposant contient dans au moins un composant un photoinitiateur en tant que constituant (g) et forme après le mélange un système à durcissement double.

8. Composition dentaire selon l'une quelconque des revendications précédentes, dans laquelle la préparation peut être un matériau de remplissage, un ciment, un agent de scellement de fissures, un matériau de prothèse, une couche de fond ou un adhésif ou un composite autoadhésif.

9. Composition dentaire selon la revendication 4, dans laquelle le système bicomposant se compose d'un premier liquide avec la composition (a), (b), (e), (f) et au moins un solvant volatile et d'un second liquide constitué de (a), (c), (d), (e), (f) et d'au moins un solvant.

10. Composition dentaire selon les revendications 4 à 9, dans laquelle chacun des deux composants est mis à disposition dans une seringue en tant que moyen d'emballage.

11. Composition dentaire selon les revendications 4 à 9, dans laquelle les deux composants sont mis à disposition dans une seringue à double chambre en tant que moyen d'emballage.

12. Composition dentaire selon la revendication 9, dans laquelle les composants de la seringue à double chambre peuvent éventuellement être mélangés de manière homogène par un mélangeur statique posé sur la seringue lors de l'évacuation.

13. Composition dentaire selon la revendication 9, dans laquelle chacun des deux composants est mis à disposition dans une bouteille en tant que moyen d'emballage.

14. Composition dentaire selon les revendications 4 à 9, dans laquelle chacun des deux composants est mis à disposition dans un moyen d'emballage différent (par exemple, seringue et bouteille).

15. Composition dentaire selon les revendications 4 à 9, dans laquelle les deux composants sont présents séparément dans un distributeur à double chambre en tant que moyen d'emballage et sont mélangés pendant l'évacuation.

16. Composition dentaire selon la revendication 4, dans laquelle le matériau est mis à disposition sous forme de poudre et de liquide, et la poudre contient les constituants (c), (d), tandis que les constituants (a), (b), (f) et éventuellement (e) sont contenus dans le liquide.

17. Composition dentaire selon les revendications 6 et 16 contenant une charge qui peut se composer d'oxydes, de nitrures, de sels métalliques inorganiques, de verre de silicate, de verre d'aluminosilicate, de verre d'aluminoborosilicate, de verre de fluoroaluminosilicate, de quartz, de dioxyde de silicium colloïdal, de dioxyde de silicium pyrogène, de dioxyde de silicium précipité, de dioxyde de silicium polymère, de dioxyde de silicium et d'oxyde de zirconium, de charges polymères, de charges composites polymérisées avec des particules inorganiques contenues et de combinaisons de ceux-ci.

18. Composition dentaire selon la revendication 17 contenant des charges provenant du groupe constitué d'oxydes, de nitrures, de sels métalliques d'au moins un élément, sélectionné parmi la série Sr, Y, Zr, Ba, La, Hf, Zn, Bi, W, des lanthanides ou des combinaisons de ceux-ci, ainsi que du verre de silicate, du verre d'aluminosilicate, du verre d'aluminoborosilicate et du verre de fluoroaluminosilicate.

19. Composition dentaire selon l'une quelconque des revendications précédentes, dans laquelle de l'eau, du méthanol, de l'éthanol, du propanol, de l'isopropanol, de l'acétone, de la méthyléthylécétone, de l'éthylèneglycol, de la glycérine et des combinaisons de ceux-ci peuvent être contenus en tant que solvant.

20. Composition dentaire selon l'une quelconque des revendications précédentes, qui contient un composé acide polymérisable avec au moins un groupe à insaturation éthylénique, sélectionné parmi le groupe des acrylates, méthacrylates ou des composés de vinyle.

21. Composition dentaire selon l'une quelconque des revendications précédentes, contenant au moins un composé d'hydroperoxyde sélectionné parmi les substances t-butylhydroperoxyde, t-amylhydroperoxyde, isopropylbenzènehydroperoxyde, p-diisopropylbenzènehydroperoxyde, 5-phényl-4-penténylhydroperoxyde, pinanhydroperoxyde, 1,1,3,3-tétraméthylbutylhydroperoxyde et des combinaisons de ceux-ci.

22. Utilisation d'une composition dentaire selon l'une quelconque des revendications précédentes 5 à 22 pour la fabrication d'une préparation pouvant être utilisée en restauration, orthodontie ou endodontie.

23. Composition dentaire selon l'une quelconque des revendications précédentes 5 à 22, qui permet un temps de durcissement après le mélange de moins de 15 minutes.

24. Composition dentaire selon l'une quelconque des revendications précédentes 5 à 23, qui permet un temps de durcissement après le mélange de moins de 5 minutes.

25. Composition dentaire selon l'une quelconque des revendications précédentes 5 à 24, qui permet un temps de durcissement après le mélange de moins de 15 minutes, après lequel les deux composants ont été stockés au moins 2 mois à 50°C.

26. Composition dentaire selon l'une quelconque des revendications précédentes 5 à 25, qui permet un temps de durcissement après le mélange de moins de 15 minutes, après lequel les deux composants ont été stockés au moins 3 mois à 37°C.
